(19)

Europäisches Patentamt
European Patent Office
Office européen des brevets

(11) **EP 3 546 591 A1**

(12) **EUROPEAN PATENT APPLICATION**
published in accordance with Art. 153(4) EPC

(43) Date of publication:
02.10.2019 Bulletin 2019/40

(21) Application number: 17874265.6

(22) Date of filing: 22.11.2017

(51) Int Cl.:
*C12Q 1/68* (2018.01)      *A61K 39/395* (2006.01)
*A61P 35/00* (2006.01)     *C07K 16/28* (2006.01)
*C12N 15/09* (2006.01)     *G01N 33/50* (2006.01)
*G01N 33/68* (2006.01)     *G01N 37/00* (2006.01)

(86) International application number:
**PCT/JP2017/041953**

(87) International publication number:
**WO 2018/097166 (31.05.2018 Gazette 2018/22)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: 24.11.2016 JP 2016228231

(71) Applicant: **Daiichi Sankyo Company, Limited
Tokyo 103-8426 (JP)**

(72) Inventors:
• **YAMADA Shinnosuke
  Tokyo 103-8426 (JP)**
• **KIYOSAWA Naoki
  Tokyo 103-8426 (JP)**

(74) Representative: **Arends, William Gerrit
Marks & Clerk LLP
15 Fetter Lane
London EC4A 1BW (GB)**

(54) **METHOD FOR PREDICTING SENSITIVITY OF CANCER TO TREATMENT WITH PD-1 IMMUNE CHECKPOINT INHIBITOR**

(57)     The present invention provides a gene set for predicting the sensitivity of a cancer to a treatment with a PD-1 immune checkpoint inhibitor. More specifically, the present invention provides one or more genes selected from the group consisting of ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B, GBP1, FAM211B, PRR5, C17orf67, FAM101B and TBL1X, and a method for predicting the sensitivity of a cancer to a treatment with a PD-1 immune checkpoint inhibitor by using the genes.

EP 3 546 591 A1

**Description**

Technical Field

The present invention relates to a method for predicting the sensitivity of a cancer to a treatment with a PD-1 immune checkpoint inhibitor and a gene signature for use in the method.

Background Art

Recently, immunotherapies have attracted attention as a new treatment for cancer. In humans, several thousands of cancer cells are presumed to be generated per day; however, the cancer cells do not always develop into cancer since these cancer cells are removed by the immune system. Usually, cancer cells and the immune system removing cancer cells are always balanced; however, it is considered that cancer is developed when the balance is lost for some reason. In killer T cells (CD8-positive T cells), which are immunocompetent cells playing a role in destroying cancer cells, programmed cell death-1 (PD-1) called an immune checkpoint is expressed. Since antigen presenting cells have programmed cell death-ligand 1 (PD-L1), which serves as a ligand for the receptors, the function of killer T cells is suppressed. However, expression of PD-L1 sometimes occurs even in cancer cells and is conceivably a mechanism why cancer cells can escape from the immune system (Non Patent Literatures 1, 2).

Recently, a medicinal agent inhibiting an immune checkpoint has been developed and highly expected as an anticancer drug having a new mechanism of action. In the results of clinical trials of an anti PD-1 antibody and an anti PD-L1 antibody and actual clinical sites, an excellent medicinal effect of the medicinal agent is exerted (Non Patent Literature 3). However, patients on whom the medicinal agent produces no therapeutic effect have been found (Non Patent Literature 3). As a biomarker predicting the sensitivity of a cancer to a treatment with an anti PD-1 antibody, for example, the expression level of PD-L1 in a tumor tissue has been investigated; however, satisfactory results have not yet been obtained (Non Patent Literature 2).

Citation List

Non Patent Literature

Non Patent Literature 1: Immunity 2013; 39(1): 1-10
Non Patent Literature 2: Cancer Discov. 2016; 6(7): 703-713
Non Patent Literature 3: N Engl J Med. 2012; 366(26): 2443-2454

Summary of Invention

The present invention provides a method for predicting the sensitivity of a cancer to a treatment with a PD-1 immune checkpoint inhibitor and a gene signature for use in the method.

The present inventors found a gene and a gene set for predicting the sensitivity of a cancer to a treatment with an immune checkpoint inhibitor. The present invention is based on the finding.

In the present invention, for example, the following inventions are provided.

(1) A method for predicting sensitivity of a cancer or a tumor of a subject to a treatment with a PD-1 immune checkpoint inhibitor, comprising measuring an expression level(s) of one or more genes selected from the group consisting of the following genes:
ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B, GBP1, FAM211B, PRR5, C17orf67, FAM101B and TBL1X in a sample obtained from the subject.

(2) The method according to the above item (1), comprising measuring expression levels of two or more genes selected from the group.

(3) The method according to the above item (1), comprising measuring an expression level(s) of at least one or more genes selected from TBL1X, FAM101B, TBC1D4, PRR5 and PTGDS.

(4) The method according to the above item (3), comprising measuring an expression level of a TBL1X gene and an expression level(s) of 1, 2, 3, 4, 5, 6, 7 or 8 or more genes selected from the group consisting of ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B, GBP1, FAM211B, PRR5, C17orf67 and FAM101B.

(5) The method according to the above item (4), comprising measuring expression levels of at least both TBL1X and FAM101B genes.

(6) The method according to the above item (5), comprising measuring expression levels of at least TBL1X, PTGDS, FAM101B, FAM211B, TBC1D4, PRR5 and C17orf67 genes.

(7) The method according to any one of the above items (1) to (6), wherein the sample obtained from the subject is a biopsy tissue sample of a cancer or a tumor, or a biopsy tissue sample of a tissue suspected as being a cancer or a tumor.

(8) The method according to the above item (7), wherein an expression level of at least PDCD1 gene is measured.

(9) The method according to any one of the above items (1) to (8), wherein the treatment with a PD-1 immune checkpoint inhibitor is administration of an anti PD-1 antibody or an anti PD-L1 antibody.

(10) A pharmaceutical composition which is the following:

    (i) a pharmaceutical composition comprising an anti PD-1 antibody or an anti PD-L1 antibody, for use in treating or preventing the cancer or the tumor in a subject having a cancer or a tumor predicted to be sensitive to a treatment with a PD-1 immune checkpoint inhibitor by the method according to the above item (1);

    (ii) a pharmaceutical composition comprising an anti PD-1 antibody or an anti PD-L1 antibody, for use in treating or preventing the cancer or the tumor in a subject having a cancer or a tumor predicted to be sensitive to a treatment with a PD-1 immune checkpoint inhibitor by the method according to the above item (2);

    (iii) a pharmaceutical composition comprising an anti PD-1 antibody or an anti PD-L1 antibody, for use in treating or preventing the cancer or the tumor in a subject having a cancer or a tumor predicted to be sensitive to a treatment with a PD-1 immune checkpoint inhibitor by the method according to the above item (3);

    (iv) a pharmaceutical composition comprising an anti PD-1 antibody or an anti PD-L1 antibody, for use in treating or preventing the cancer or the tumor in a subject having a cancer or a tumor predicted to be sensitive to a treatment with a PD-1 immune checkpoint inhibitor by the method according to the above item (4);

    (v) a pharmaceutical composition comprising an anti PD-1 antibody or an anti PD-L1 antibody, for use in treating or preventing the cancer or the tumor in a subject having a cancer or a tumor predicted to be sensitive to a treatment with a PD-1 immune checkpoint inhibitor by the method according to the above item (5);

    (vi) a pharmaceutical composition comprising an anti PD-1 antibody or an anti PD-L1 antibody, for use in treating or preventing the cancer or the tumor in a subject having a cancer or a tumor predicted to be sensitive to a treatment with a PD-1 immune checkpoint inhibitor by the method according to the above item (6);

    (vii) a pharmaceutical composition comprising an anti PD-1 antibody or an anti PD-L1 antibody, for use in treating or preventing the cancer or the tumor in a subject having a cancer or a tumor predicted to be sensitive to a treatment with a PD-1 immune checkpoint inhibitor by the method according to the above item (7);

    (viii) a pharmaceutical composition comprising an anti PD-1 antibody or an anti PD-L1 antibody, for use in treating or preventing the cancer or the tumor in a subject having a cancer or a tumor predicted to be sensitive to a treatment with a PD-1 immune checkpoint inhibitor by the method according to the above item (8) ; or

    (ix) a pharmaceutical composition comprising an anti PD-1 antibody or an anti PD-L1 antibody, for use in treating or preventing the cancer or the tumor in a subject having a cancer or a tumor predicted to be sensitive to a treatment with a PD-1 immune checkpoint inhibitor by the method according to the above item (9).

(11) A gene signature comprising one or more genes selected from the group consisting of ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B, GBP1, FAM211B, PRR5, C17orf67, FAM101B and TBL1X, for use in predicting whether or not a cancer or a tumor is sensitive to a treatment with a PD-1 immune checkpoint inhibitor.

(12) The gene signature according to the above item (11), comprising at least one or more genes selected from TBL1X, FAM101B, TBC1D4, PRR5 and PTGDS.

(13) The gene signature according to the above item (12), comprising at least TBL1X and FAM101B.

(14) The gene signature according to the above item (13), comprising at least TBL1X, PTGDS, FAM101B, FAM211B, TBC1D4, PRR5 and C17orf67.

(15) A combination of nucleic acid probes or primer sets for a plurality of genes selected from the group consisting of ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B, GBP1, FAM211B, PRR5, C17orf67, FAM101B and TBL1X.

(16) The combination of nucleic acid probes or primer sets according to the above item (15), wherein the plurality of genes comprises at least one or more genes selected from TBL1X, FAM101B, TBC1D4, PRR5 and PTGDS.

(17) The combination of nucleic acid probes or primer sets according to the above item (16), wherein the plurality of genes comprises at least TBL1X, PTGDS, FAM101B, FAM211B, TBC1D4, PRR5 and C17orf67.

(18) A microarray comprising the nucleic acid probes defined in any one of the above items (15) to (17).

(19) A kit for use in determining sensitivity to a treatment with a PD-1 immune checkpoint inhibitor, comprising means for measuring an expression level(s) of one or more genes selected from the group consisting of ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B, GBP1, FAM211B, PRR5, C17orf67, FAM101B and TBL1X.

(20) The kit according to the above item (19), wherein the one or more genes comprise at least one or more genes selected from TBL1X, FAM101B, TBC1D4, PRR5 and PTGDS.

(21) The kit according to the above item (20), wherein the one or more genes comprise at least TBL1X, PTGDS, FAM101B, FAM211B, TBC1D4, PRR5 and C17orf67.

In the present invention, it is possible to predict the sensitivity to a treatment with a PD-1 immune checkpoint inhibitor by measuring expression of a gene signature. In the present invention, it is particularly possible to predict the sensitivity of a subject to a treatment with a PD-1 immune checkpoint inhibitor (for example, anti PD-1 antibody or anti PD-L1 antibody) by measuring expression of a gene signature in a biopsy tissue sample. In the present invention, it is also possible to administer a PD-1 immune checkpoint inhibitor (for example, anti PD-1 antibody or anti PD-L1 antibody) to a subject having sensitivity to or a subject evaluated as having sensitivity to a treatment with a PD-1 immune checkpoint inhibitor.

Brief Description of Drawings

Figure 1 shows the relationship between the gene signature score by using gene signature A and the sensitivity to an anti PD-1 antibody.

Figure 2 shows the relationship between the gene signature score by using gene signature A + B and the sensitivity to an anti PD-1 antibody.

Figure 3 shows the relationship between the gene signature score by using gene signature A and the gene signature score by using gene signature A + B, where scores of the same patient are connected by a line.

Figure 4A shows the relationship between the expression levels of TBL1X in cancers and the therapeutic effect by an anti PD-1 antibody serving as an immune checkpoint inhibitor against various cancers.

Figure 4B shows the relationship between the expression levels of FAM101B in cancers and the therapeutic effect by an anti PD-1 antibody serving as an immune checkpoint inhibitor against various cancers.

Figure 4C shows the relationship between the expression level of TBC1D4 in cancers and the therapeutic effect by an anti PD-1 antibody serving as an immune checkpoint inhibitor against various cancers.

Figure 4D shows the relationship between the expression level of PRR5 in cancers and the therapeutic effect by an anti PD-1 antibody serving as an immune checkpoint inhibitor against various cancers.

Figure 4E shows the relationship between the expression level of PTGDS in cancers and the therapeutic effect by an anti PD-1 antibody serving as an immune checkpoint inhibitor against various cancers.

Figure 4F shows the relationship between the expression level of FAM211B in cancers and the therapeutic effect by an anti PD-1 antibody serving as an immune checkpoint inhibitor against various cancers.

Figure 4G shows the relationship between the expression level of RAB11FIP5 in cancers and the therapeutic effect by an anti PD-1 antibody serving as an immune checkpoint inhibitor against various cancers.

Figure 4H shows the relationship between the expression level of GCNT2 in cancers and the therapeutic effect by an anti PD-1 antibody serving as an immune checkpoint inhibitor against various cancers.

Figure 4I shows the relationship between the expression level of TSPAN32 in cancers and the therapeutic effect by an anti PD-1 antibody serving as an immune checkpoint inhibitor against various cancers.

Figure 4J shows the relationship between the expression level of MYB in cancers and the therapeutic effect by an anti PD-1 antibody serving as an immune checkpoint inhibitor against various cancers.

Figure 4K shows the relationship between the expression level of ADAM8 in cancers and the therapeutic effect by an anti PD-1 antibody serving as an immune checkpoint inhibitor against various cancers.

Figure 4L shows the relationship between the expression level of ASAP1 in cancers and the therapeutic effect by an anti PD-1 antibody serving as an immune checkpoint inhibitor against various cancers.

Figure 4M shows the relationship between the expression level of ITGB1 in cancers and the therapeutic effect by an anti PD-1 antibody serving as an immune checkpoint inhibitor against various cancers.

Figure 4N shows the relationship between the expression level of MBOAT1 in cancers and the therapeutic effect by an anti PD-1 antibody serving as an immune checkpoint inhibitor against various cancers.

Figure 4O shows the relationship between the expression level of ASB2 in cancers and the therapeutic effect by an anti PD-1 antibody serving as an immune checkpoint inhibitor against various cancers.

Figure 4P shows the relationship between the expression level of COTL1 in cancers and the therapeutic effect by an anti PD-1 antibody serving as an immune checkpoint inhibitor against various cancers.

Figure 4Q shows the relationship between the expression level of SGPP2 in cancers and the therapeutic effect by an anti PD-1 antibody serving as an immune checkpoint inhibitor against various cancers.

Figure 4R shows the relationship between the expression level of FAM65B in cancers and the therapeutic effect by an anti PD-1 antibody serving as an immune checkpoint inhibitor against various cancers.

Figure 4S shows the relationship between the expression level of SLC4A8 in cancers and the therapeutic effect by an anti PD-1 antibody serving as an immune checkpoint inhibitor against various cancers.

Figure 4T shows the relationship between the expression level of LEF1-AS1 in cancers and the therapeutic effect by an anti PD-1 antibody serving as an immune checkpoint inhibitor against various cancers.

Figure 4U shows the relationship between the expression level of RXRA in cancers and the therapeutic effect by an anti PD-1 antibody serving as an immune checkpoint inhibitor against various cancers.

Figure 4V shows the relationship between the expression level of NIN in cancers and the therapeutic effect by an anti PD-1 antibody serving as an immune checkpoint inhibitor against various cancers.

Figure 4W shows the relationship between the expression level of VCL in cancers and the therapeutic effect by an anti PD-1 antibody serving as an immune checkpoint inhibitor against various cancers.

Figure 4X shows the relationship between the expression level of KLRD1 in cancers and the therapeutic effect by an anti PD-1 antibody serving as an immune checkpoint inhibitor against various cancers.

Figure 4Y shows the relationship between the expression level of EMP3 in cancers and the therapeutic effect by an anti PD-1 antibody serving as an immune checkpoint inhibitor against various cancers.

Figure 4Z shows the relationship between the expression level of PTPN13 in cancers and the therapeutic effect by an anti PD-1 antibody serving as an immune checkpoint inhibitor against various cancers.

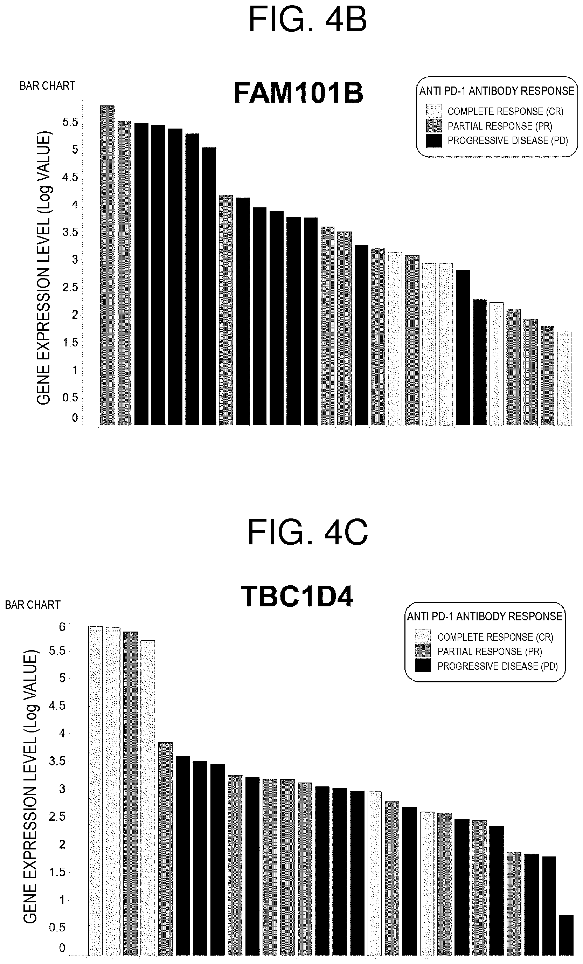

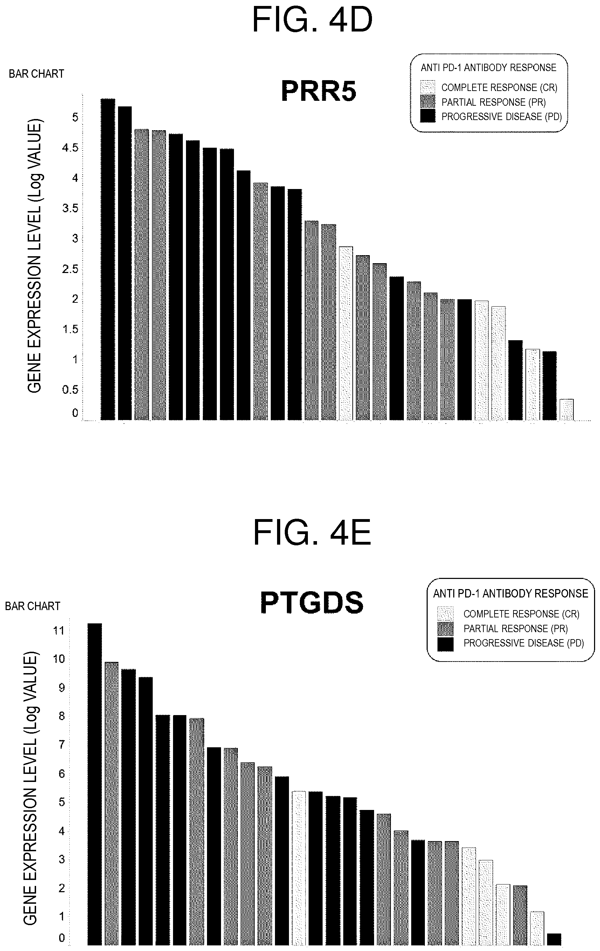

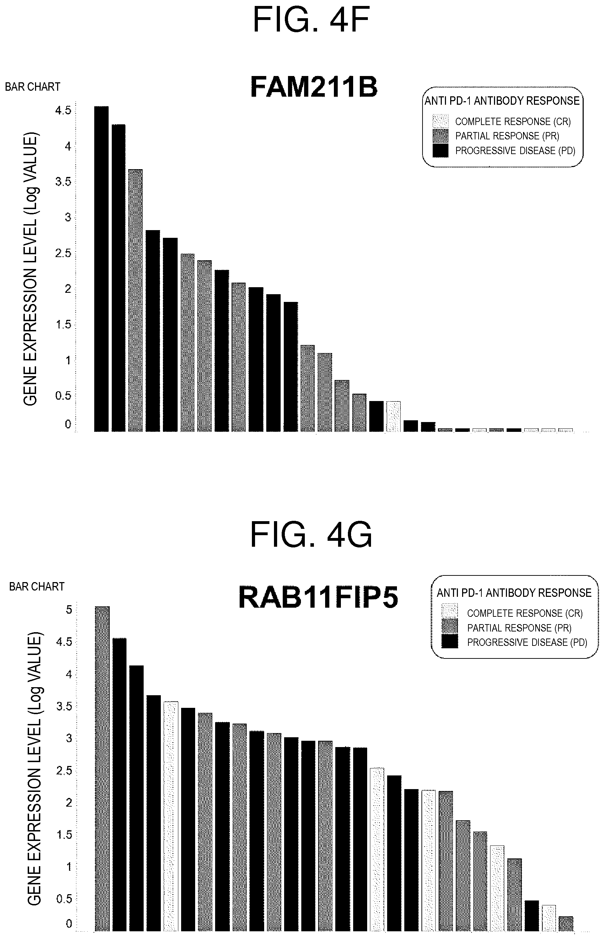

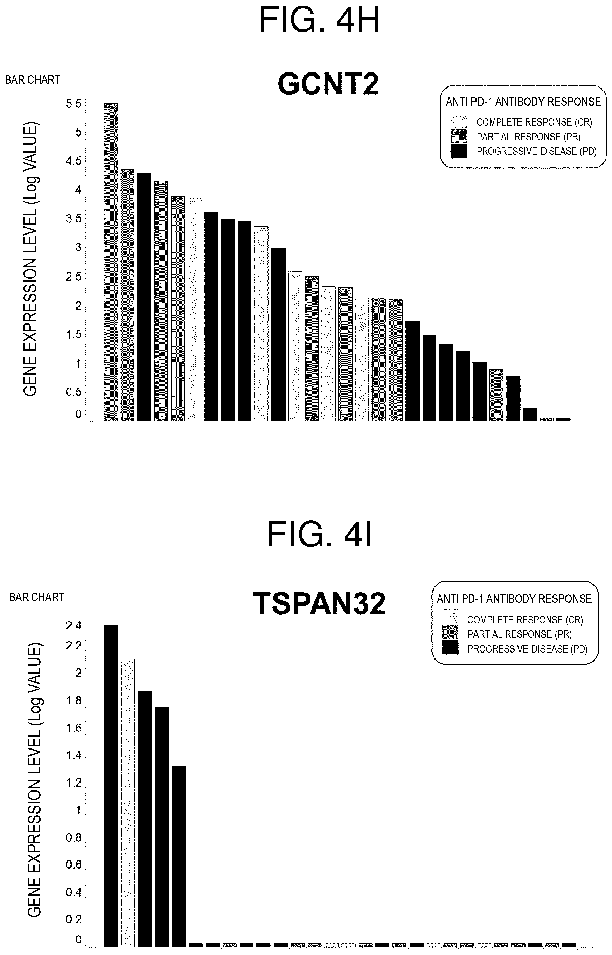

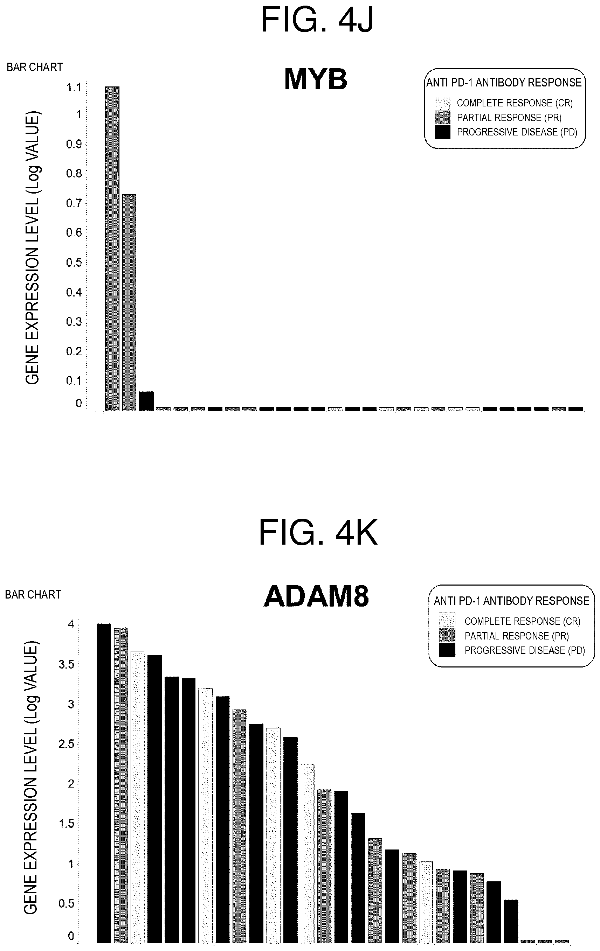

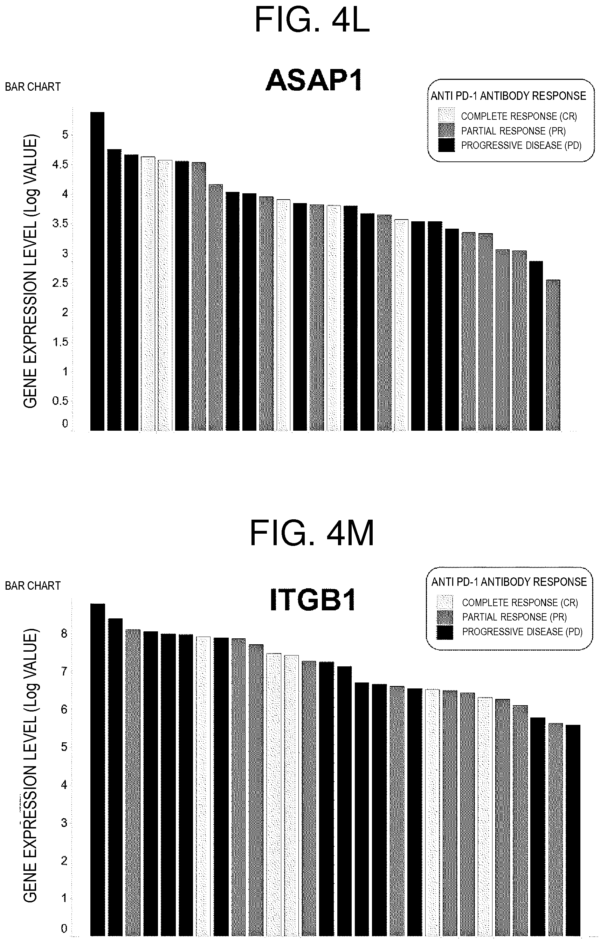

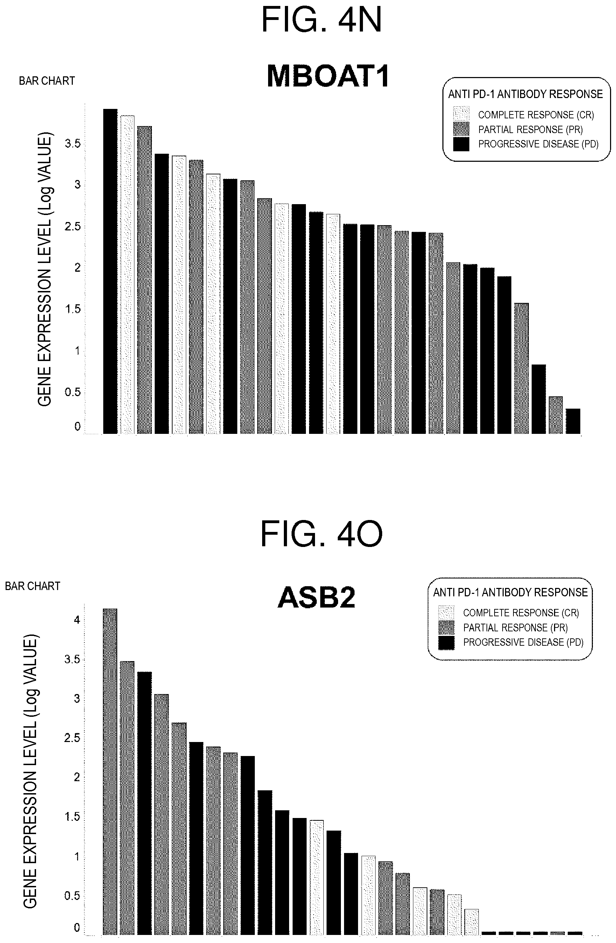

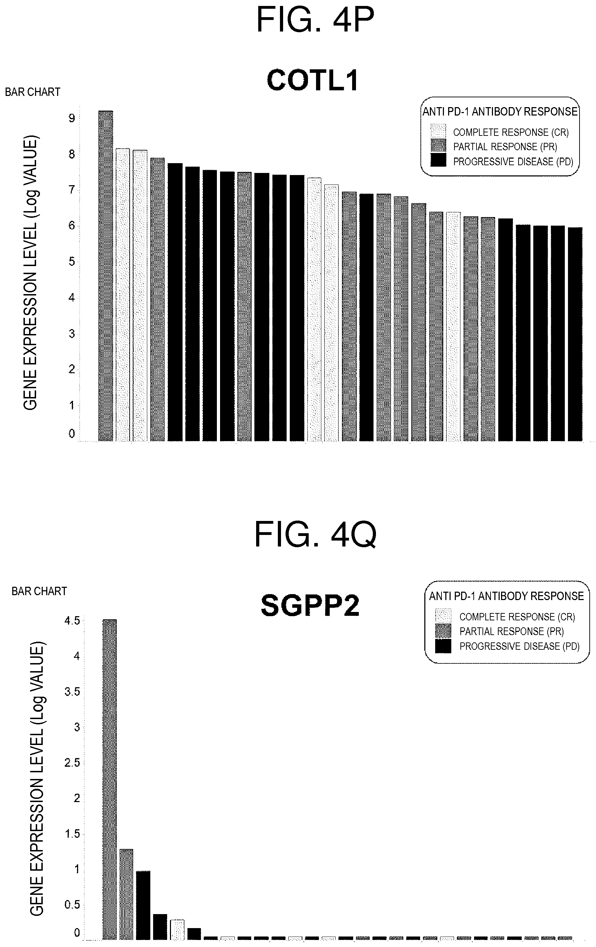

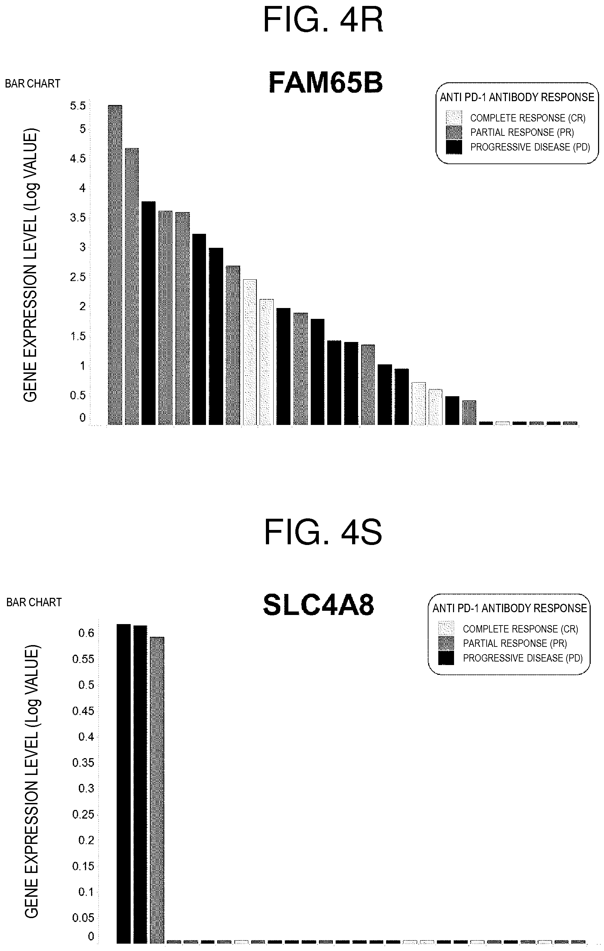

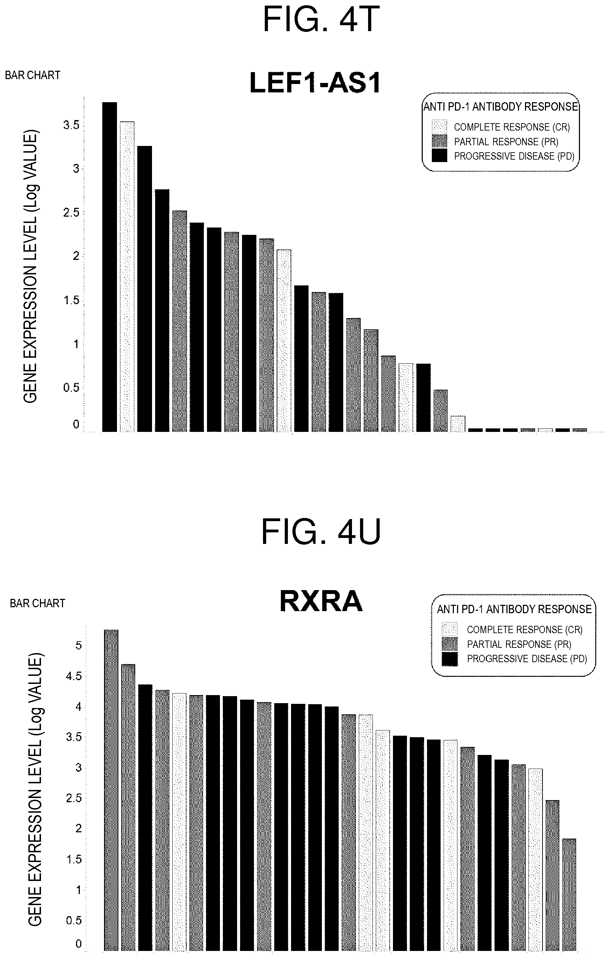

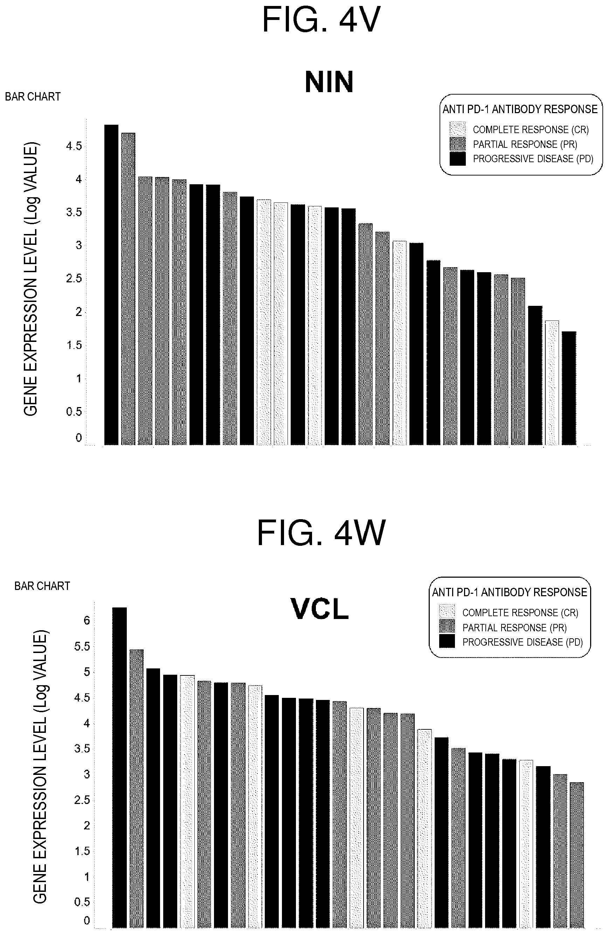

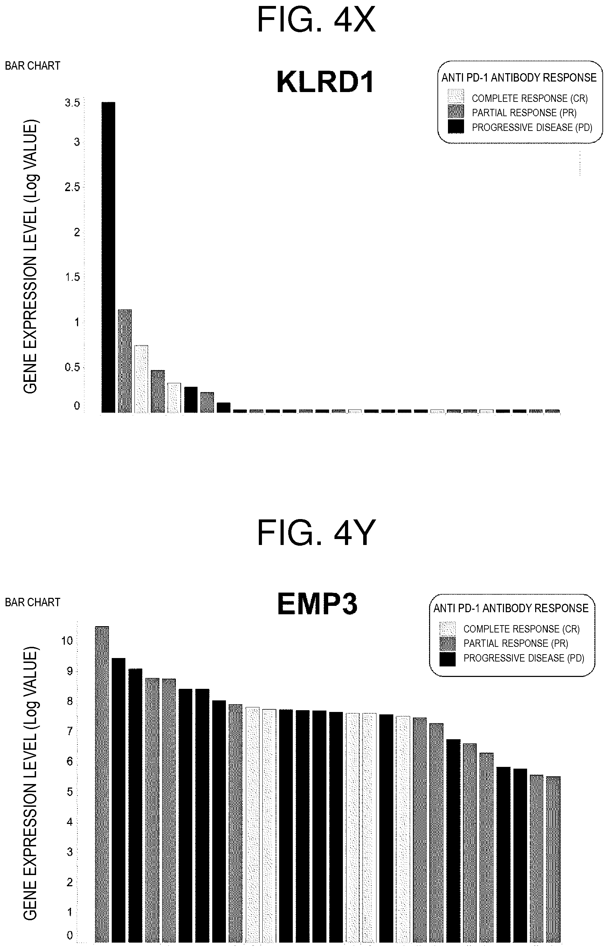

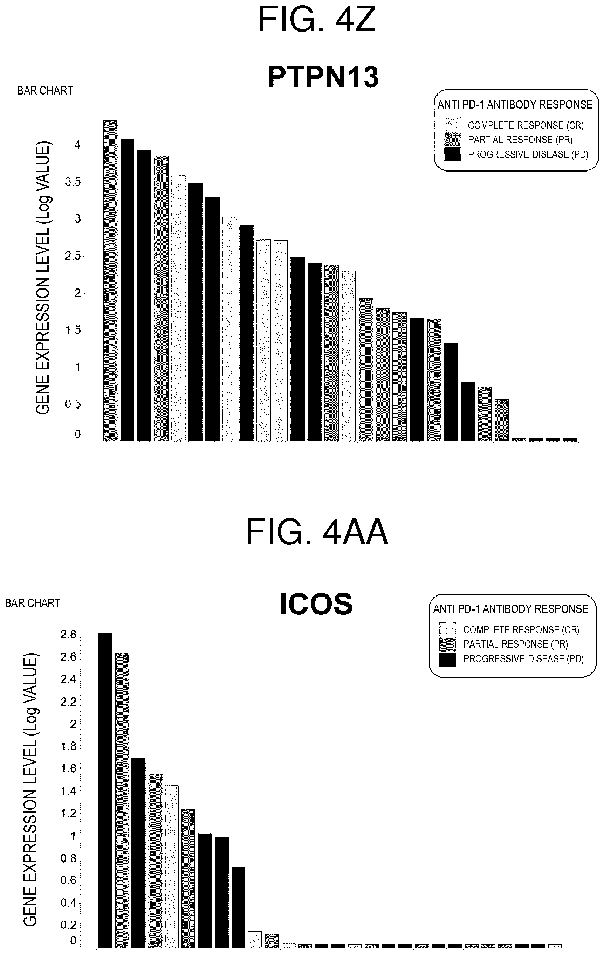

Figure 4AA shows the relationship between the expression level of ICOS in cancers and the therapeutic effect by an anti PD-1 antibody serving as an immune checkpoint inhibitor against various cancers.

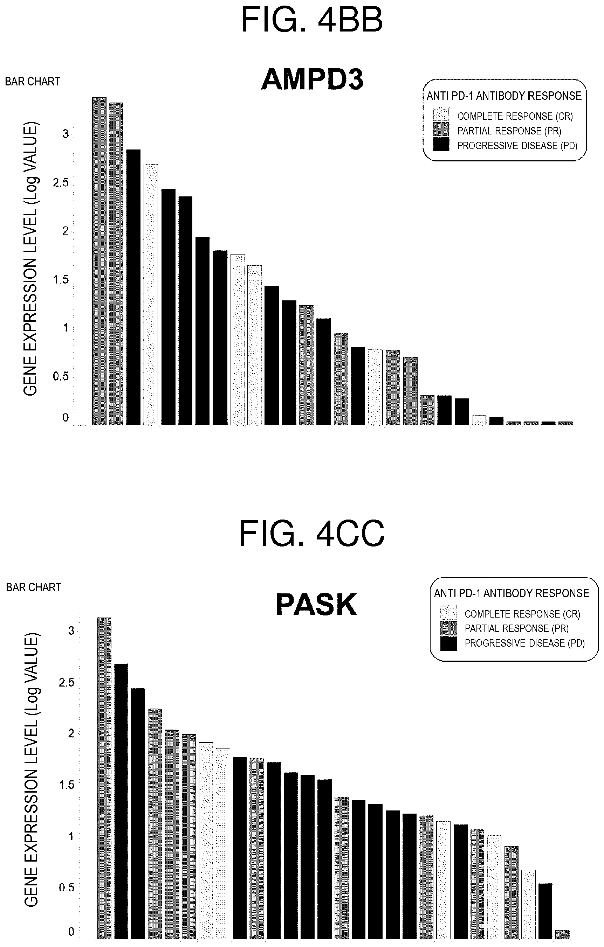

Figure 4BB shows the relationship between the expression level of AMPD3 in cancers and the therapeutic effect by an anti PD-1 antibody serving as an immune checkpoint inhibitor against various cancers.

Figure 4CC shows the relationship between the expression level of PASK in cancers and the therapeutic effect by an anti PD-1 antibody serving as an immune checkpoint inhibitor against various cancers.

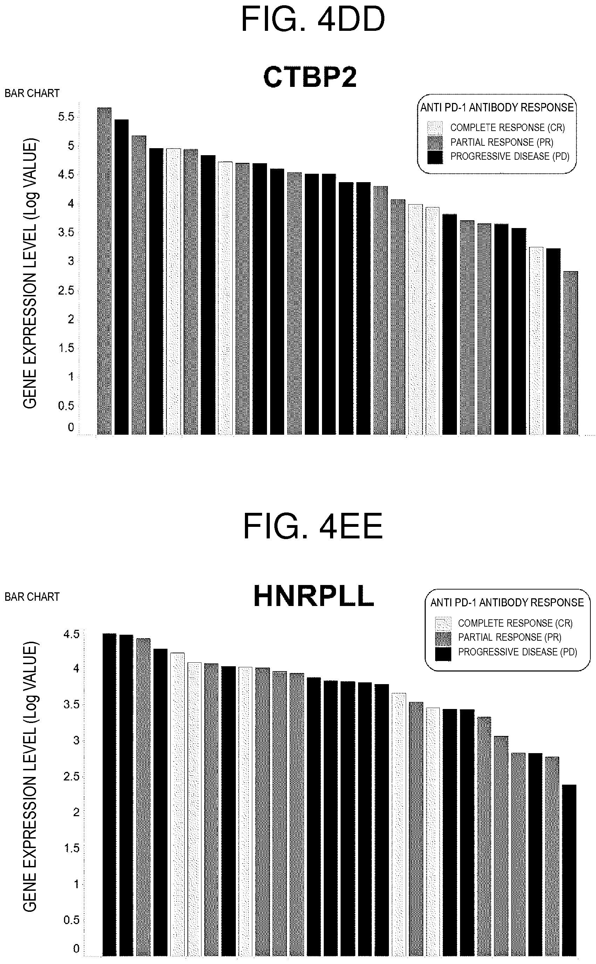

Figure 4DD shows the relationship between the expression level of CTBP2 in cancers and the therapeutic effect by an anti PD-1 antibody serving as an immune checkpoint inhibitor against various cancers.

Figure 4EE shows the relationship between the expression level of HNRPLL in cancers and the therapeutic effect by an anti PD-1 antibody serving as an immune checkpoint inhibitor against various cancers.

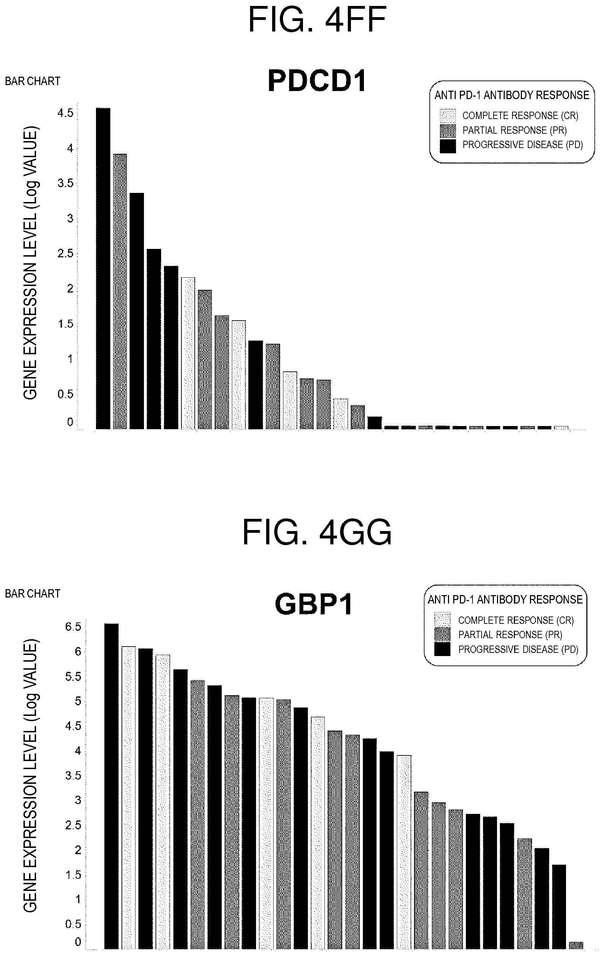

Figure 4FF shows the relationship between the expression level of PDCD1 in cancers and the therapeutic effect by an anti PD-1 antibody serving as an immune checkpoint inhibitor against various cancers.

Figure 4GG shows the relationship between the expression level of GBP1 in cancers and the therapeutic effect by an anti PD-1 antibody serving as an immune checkpoint inhibitor against various cancers.

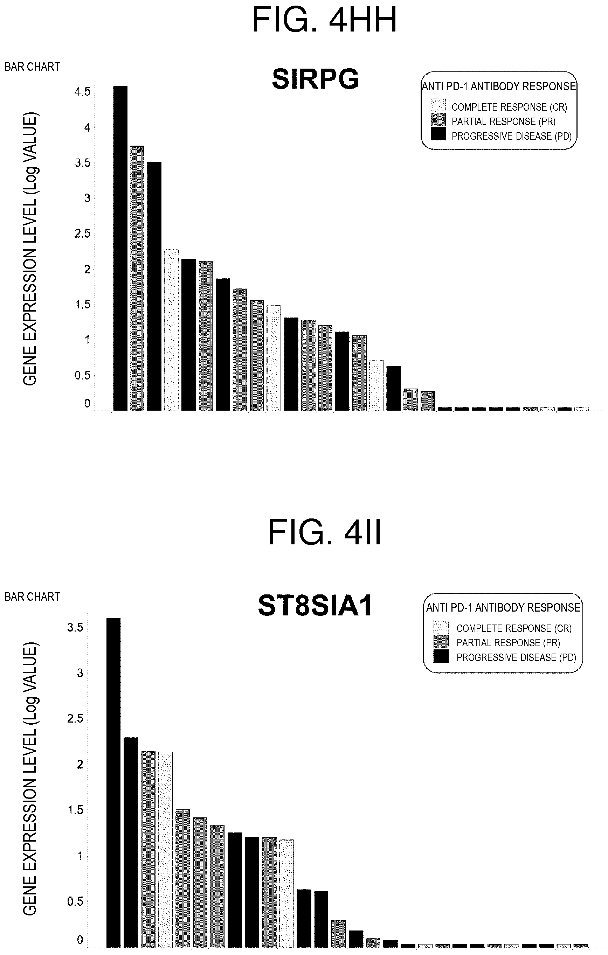

Figure 4HH shows the relationship between the expression level of SIRPG in cancers and the therapeutic effect by an anti PD-1 antibody serving as an immune checkpoint inhibitor against various cancers.

Figure 4II shows the relationship between the expression level of ST8SIA1 in cancers and the therapeutic effect by an anti PD-1 antibody serving as an immune checkpoint inhibitor against various cancers.

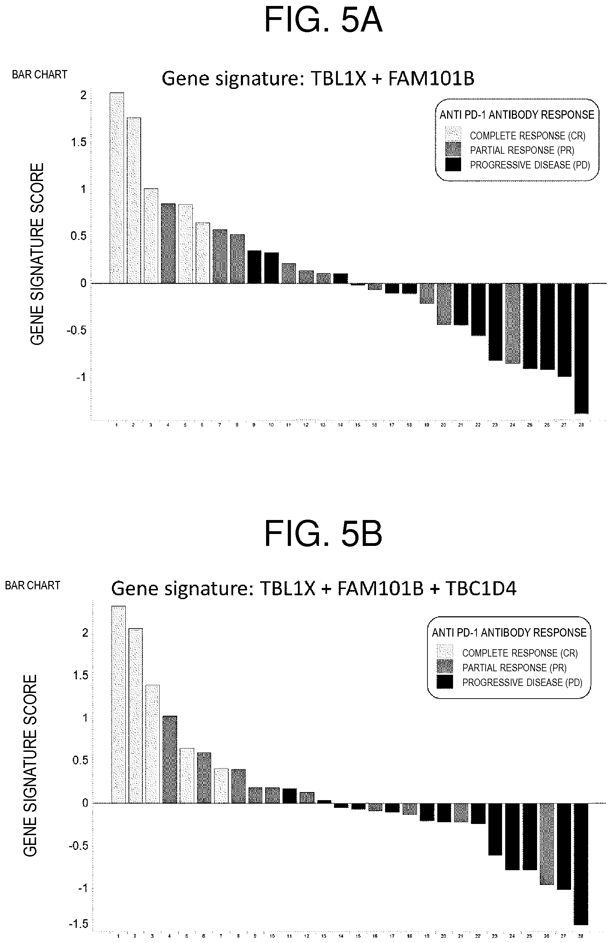

Figure 5A shows the relationship between the gene signature scores based on expression of TBL1X and FAM101B genes and the therapeutic effect by an anti PD-1 antibody serving as an immune checkpoint inhibitor against various cancers.

Figure 5B shows the relationship between the gene signature scores based on expression of TBL1X, FAM101B and TBC1D4 genes and the therapeutic effect by an anti PD-1 antibody serving as an immune checkpoint inhibitor against various cancers.

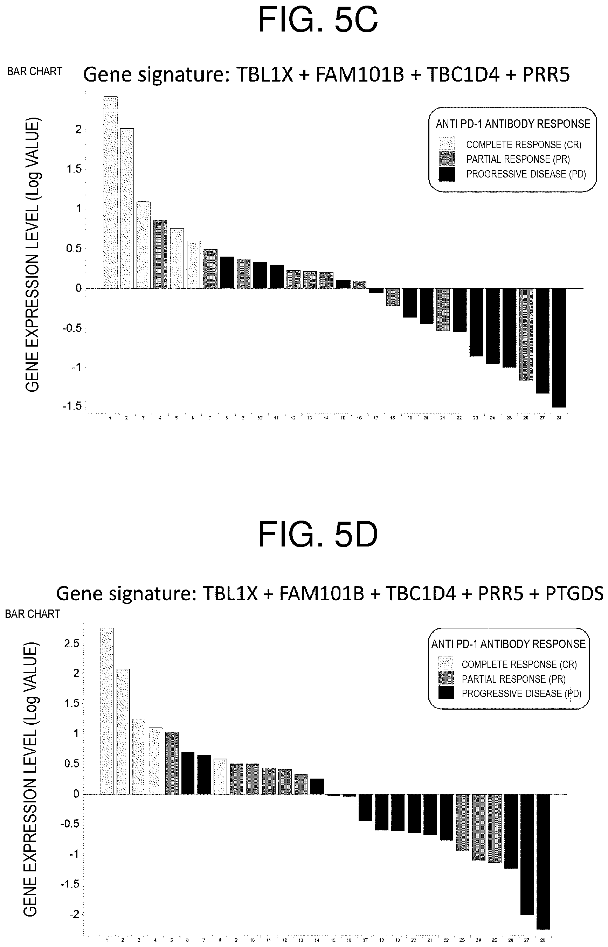

Figure 5C shows the relationship between the gene signature scores based on expression of TBL1X, FAM101B, TBC1D4 and PRR5 genes and the therapeutic effect by an anti PD-1 antibody serving as an immune checkpoint inhibitor against various cancers.

Figure 5D shows the relationship between the gene signature scores based on expression of TBL1X, FAM101B, TBC1D4, PRR5 and PTGDS genes and the therapeutic effect by an anti PD-1 antibody serving as an immune checkpoint inhibitor against various cancers.

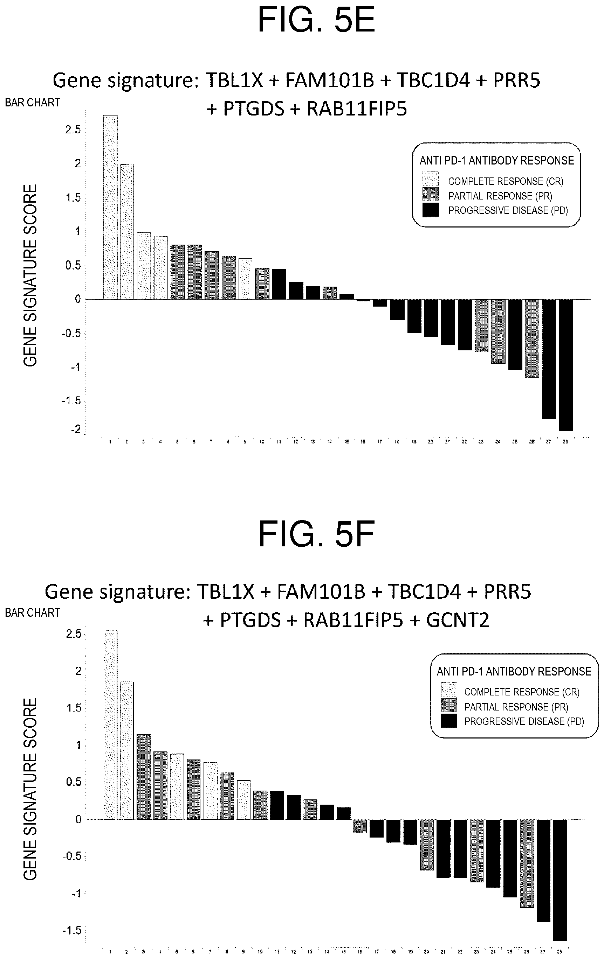

Figure 5E shows the relationship between the gene signature scores based on expression of TBL1X, FAM101B, TBC1D4, PRR5, PTGDS and RAB11FIP5 genes and the therapeutic effect by an anti PD-1 antibody serving as an immune checkpoint inhibitor against various cancers.

Figure 5F shows the relationship between the gene signature scores based on expression of TBL1X, FAM101B, TBC1D4, PRR5, PTGDS, RAB11FIP5 and GCNT2 genes and the therapeutic effect by an anti PD-1 antibody serving as an immune checkpoint inhibitor against various cancers.

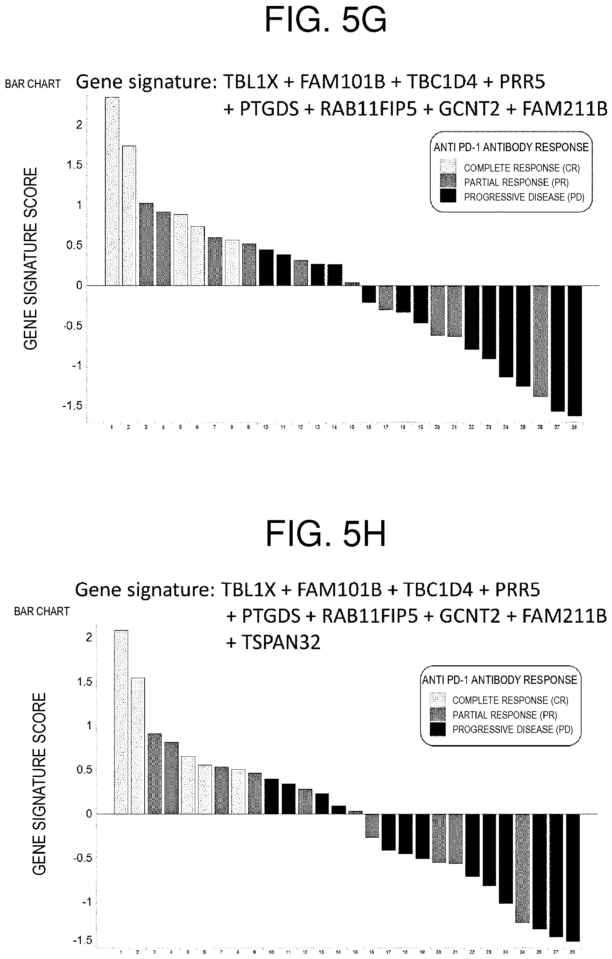

Figure 5G shows the relationship between the gene signature scores based on expression of TBL1X, FAM101B, TBC1D4, PRR5, PTGDS, RAB11FIP5, GCNT2 and FAM211B genes and the therapeutic effect by an anti PD-1 antibody serving as an immune checkpoint inhibitor against various cancers.

Figure 5H shows the relationship between the gene signature scores based on expression of TBL1X, FAM101B, TBC1D4, PRR5, PTGDS, RAB11FIP5, GCNT2, FAM211B and TSPAN32 genes and the therapeutic effect by an anti PD-1 antibody serving as an immune checkpoint inhibitor against various cancers.

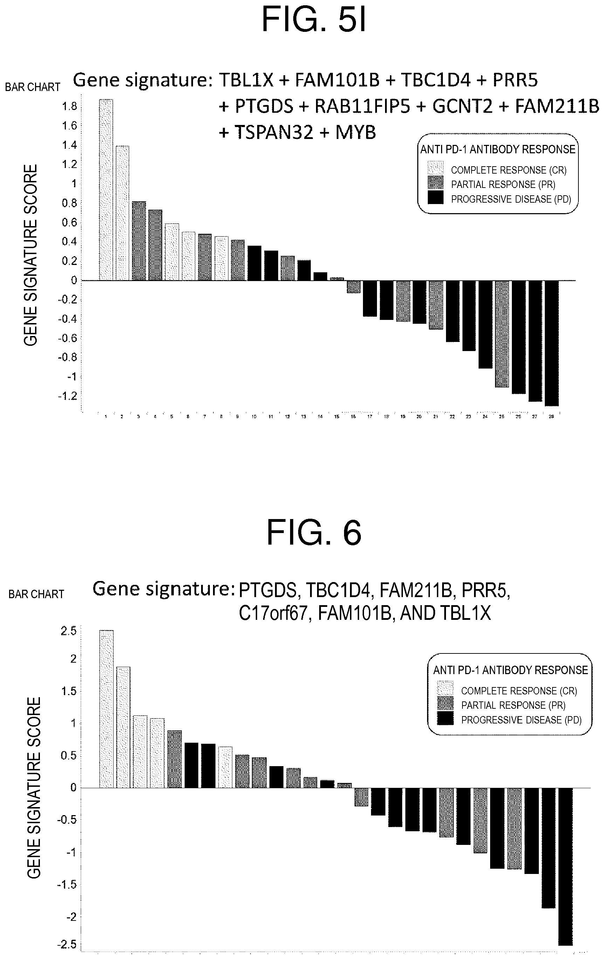

Figure 5I shows the relationship between the gene signature scores based on expression of TBL1X, FAM101B, TBC1D4, PRR5, PTGDS, RAB11FIP5, GCNT2, FAM211B, TSPAN32 and MYB genes and the therapeutic effect by an anti PD-1 antibody serving as an immune checkpoint inhibitor against various cancers.

Figure 6 shows the relationship between the gene signature scores based on expression of PTGDS, TBC1D4, FAM211B, PRR5, C17orf67, FAM101B and TBL1X genes and the therapeutic effect by an anti PD-1 antibody serving as an immune checkpoint inhibitor against various cancers.

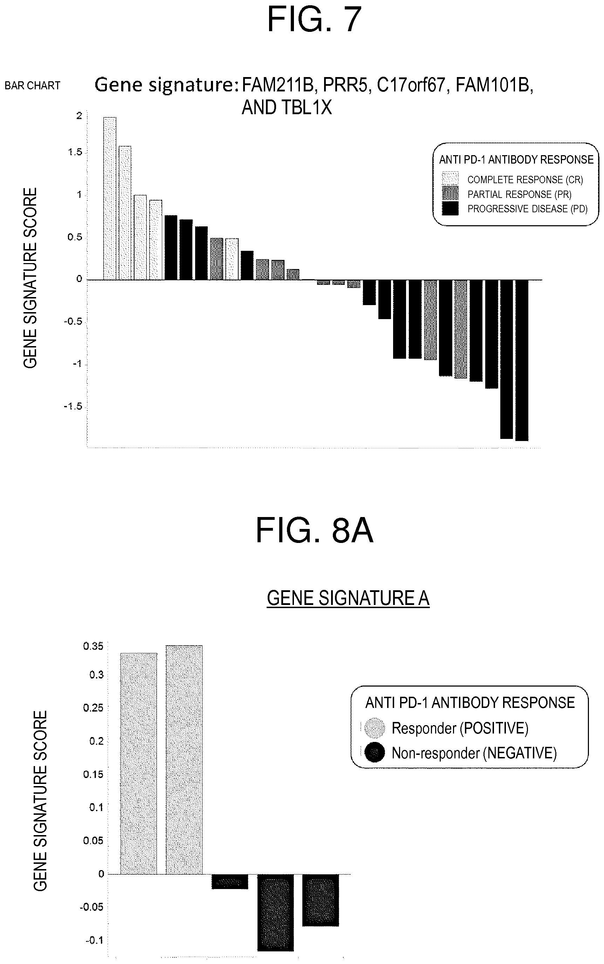

Figure 7 shows the relationship between the gene signature scores based on expression of FAM211B, PRR5, C17orf67, FAM101B and TBL1X genes and the therapeutic effect by an anti PD-1 antibody serving as an immune checkpoint inhibitor against various cancers.

Figure 8A shows the relationship between gene signature scores calculated from gene signature A in a biopsy tissue sample of a patient before a treatment with an anti PD-1 antibody serving as an immune checkpoint inhibitor and the sensitivity to the medicinal agent.

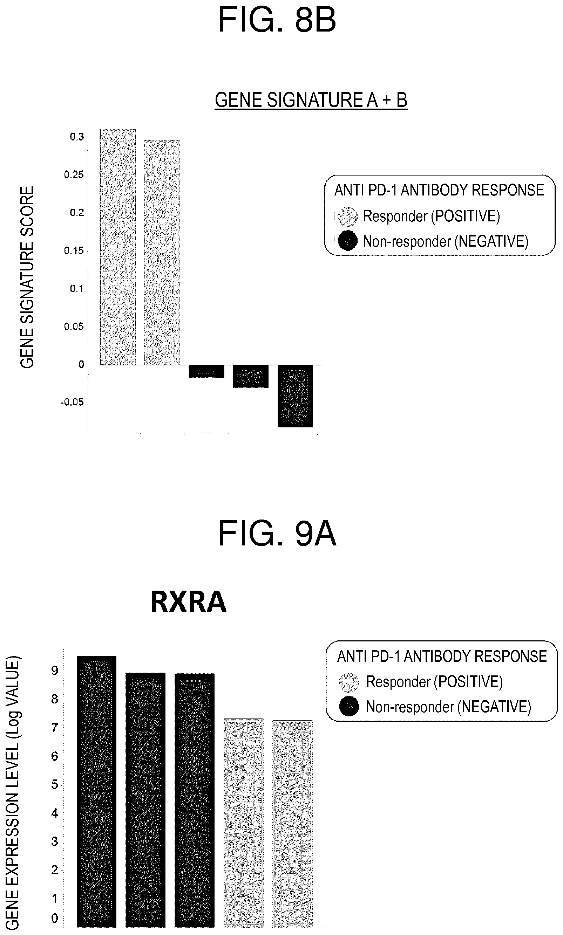

Figure 8B shows the relationship between gene signature scores calculated from gene signature A + B in a biopsy tissue sample of a patient before a treatment with an anti PD-1 antibody serving as an immune checkpoint inhibitor and the sensitivity to the medicinal agent.

Figure 9A shows the relationship between the expression level of an RXRA gene in cancers and the sensitivity of the cancers to a treatment with an anti PD-1 antibody serving as an immune checkpoint inhibitor.

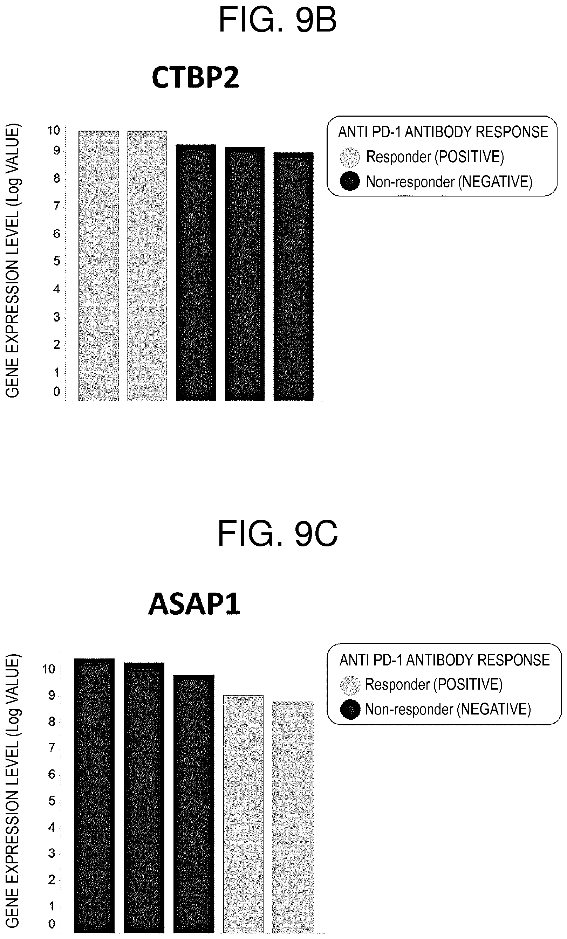

Figure 9B shows the relationship between the expression level of a CTBP2 gene in cancers and the sensitivity of the cancers to a treatment with an anti PD-1 antibody serving as an immune checkpoint inhibitor.

Figure 9C shows the relationship between the expression level of an ASAP1 gene in cancers and the sensitivity of the cancers to a treatment with an anti PD-1 antibody serving as an immune checkpoint inhibitor.

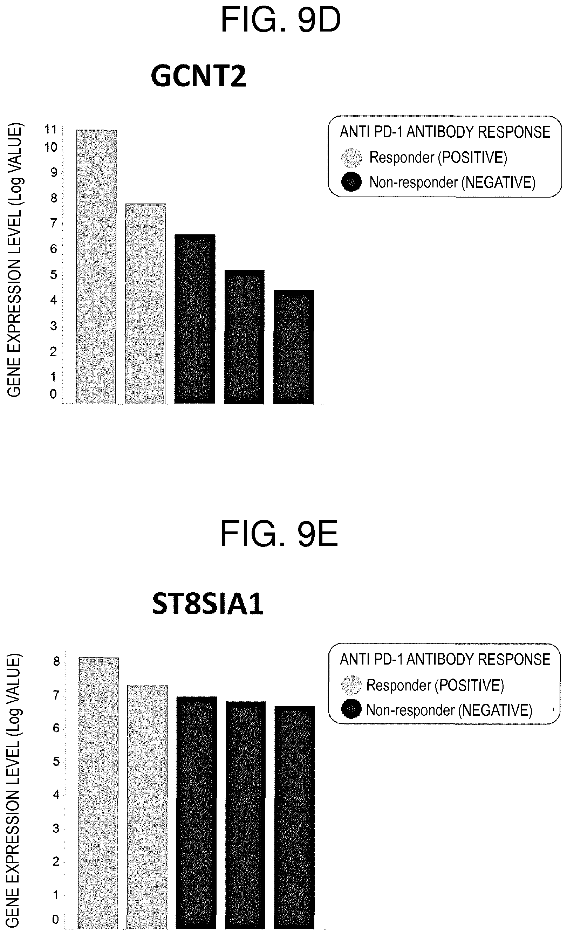

Figure 9D shows the relationship between the expression level of a GCNT2 gene in cancers and the sensitivity of the cancers to a treatment with an anti PD-1 antibody serving as an immune checkpoint inhibitor.

Figure 9E shows the relationship between the expression level of an ST8SIA1 gene in cancers and the sensitivity of the cancers to a treatment with an anti PD-1 antibody serving as an immune checkpoint inhibitor.

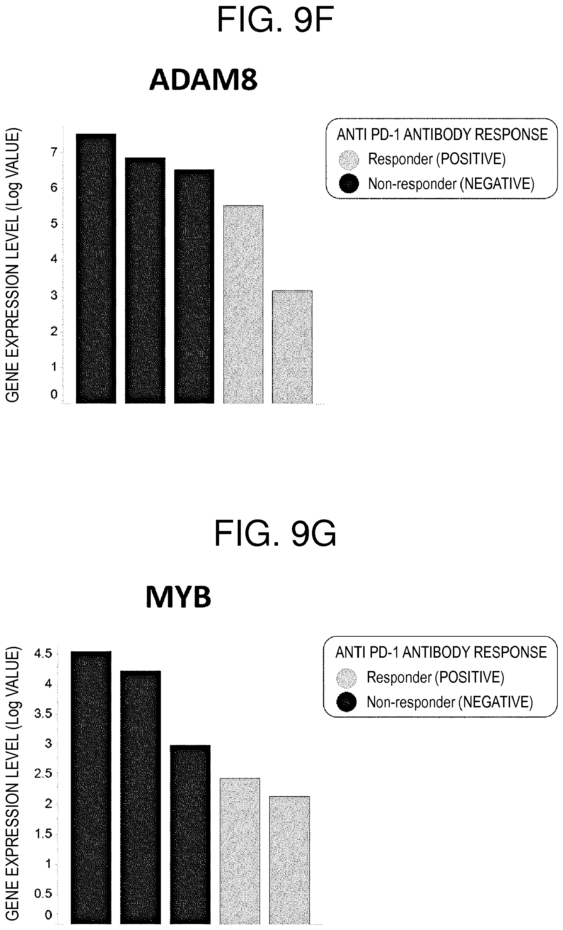

Figure 9F shows the relationship between the expression level of an ADAM8 gene in cancers and the sensitivity of the cancers to a treatment with an anti PD-1 antibody serving as an immune checkpoint inhibitor.

Figure 9G shows the relationship between the expression level of an MYB gene in cancers and the sensitivity of the cancers to a treatment with an anti PD-1 antibody serving as an immune checkpoint inhibitor.

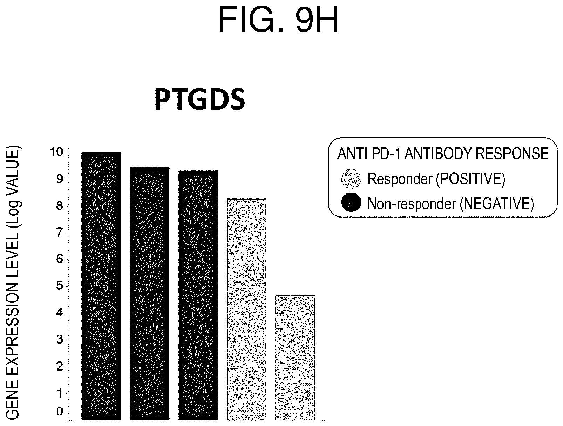

Figure 9H shows the relationship between the expression level of a PTGDS gene in cancers and the sensitivity of the cancers to a treatment with an anti PD-1 antibody serving as an immune checkpoint inhibitor.

Description of Embodiments

In the specification, the "subject" is a mammal, particularly a human.

In the specification, the "cancer" refers to a malignant tumor, particularly, a tumor having invasiveness and malignant natures such as proliferative property and metastatic property. In the specification, the "tumor" refers to a mass of a tissue autonomously and excessively growing in living bodies. In the present invention, a malignant tumor, a cancer, a malignant neoplasm, a carcinoma, a sarcoma and the like will be sometimes collectively referred to as "cancer."

In the specification, the "gene signature" refers to a single gene or a combination of genes, more specifically, a gene or a combination of genes for use in predicting the sensitivity of the cancer or the tumor to a predetermined treatment by measuring the expression level thereof in a cancer or a tumor of a subject.

In the specification, the "expression level" refers to the amount of messenger RNA (hereinafter referred to as "mRNA") of a gene or the amount of protein encoded by a gene.

In the specification, the phrase "sensitivity is high" means that "treatment with a PD-1 immune checkpoint inhibitor" is effective. The phrase "sensitivity is high" has the same meaning as the phrase "tolerability or resistance is low." These phrases can be interchangeably used. In the specification, the phrase "sensitivity is low" has the same meaning as the phrase "tolerability or resistance is high." These phrases can be interchangeably used.

In the specification, the "complete response" in the case of a solid cancer refers to the "CR" defined in the immune related response criteria: irRECIST (Bohnsack O. et al., Ann. Oncl., 25 (Supplement 4): 361-372, 2014). In the specification, the "partial response" in the case of a solid cancer refers to "PR" defined in the same guidelines as above. In the specification, "stable disease" in the case of a solid cancer refers to "SD" defined in the same guidelines. In the specification, "progressive disease" in the case of a solid cancer refers to a "PD" defined in the same guidelines.

In the specification, the "treatment" includes delaying, terminating and suppressing of progression of a disease, and regression and disappearance of a disease site.

In the specification, the "prevention" includes suppressing onset of a disease and reducing the possibility of onset.

In the specification, the "ligand" refers to a substance that specifically binds to a predetermined receptor. For example, the "ligand for PD-1" refers to a substance that specifically binds to PD-1, such as PD-L1 or PD-L2.

In the specification, the "PD-1," which is referred to as programmed cell death-1 (PDCD1), is a receptor belonging to a B7/CD28 superfamily and expressing on the surface of T cell membrane. When PD-1 interacts with its ligand (PD-L1 or PD-L2, particularly PD-L1), the action of T cells is suppressed. It is known that when PD-L1 is expressed by cancer cells and tumor cells, the action of T cells (for example, killer T cells) expressing PD-1 is suppressed, with the result that cancer cells and tumor cells escape from immunity. In the specification, a mechanism of suppressing the action of T cells expressing PD-1 by PD-L1 is referred to as the "PD-1 immune checkpoint" and an inhibitor against suppression of the action refers to a "PD-1 immune checkpoint inhibitor." Clinical trials using, e.g., an anti PD-1 antibody show that a cancer and a tumor can be treated by a treatment with such an immune checkpoint inhibitor (for example, an agent of reducing interaction between PD-1 and PD-L1, and for example, an anti PD-1 antibody or an anti PD-L1 antibody).

As to PD-1 in a human, the sequence of mRNA thereof is registered, for example, in the GenBank under NCBI Reference Sequence: NM_005018.2. The amino acid sequence of PD-1 is registered in the GenBank under NCBI Reference Sequence: NP_005009.2. As to PD-L1 in a human, the nucleotide sequence of the gene region of PD-L1 is registered, for example, in the GenBank under NCBI Reference Sequence: NC_000009.12. As to isoform a, the sequence of mRNA thereof is registered under NM_014143.3 and the amino acid sequence of a protein thereof is registered under NP_054862.1. As to isoform b, the sequence of mRNA thereof is registered under NM_001267706.1 and the amino acid sequence of a protein thereof is registered under NP_001254635.1. As to isoform c, the sequence of mRNA thereof is registered under NM_001314029.1 and the amino acid sequence of a protein thereof is registered under NP_001300958.1.

In the specification, the "combination" refers to a combination of a plurality of components. In the combination, individual components may be present all in a discrete form or a mixed form.

In the specification, the "nucleic acid probe" refers to a single stranded nucleic acid that can be hybridized with a predetermined nucleic acid for detecting or quantifying the nucleic acid.

The nucleic acid probe is fixed onto the surface of a support such as an array or a microarray, and thereby, a predetermined nucleic acid can be immobilized to the support holding the nucleic acid probe. The nucleic acid immobilized can be detected or quantified with the help of a label attached to the nucleic acid.

The nucleic acid probe can be used in quantitative PCR. In the quantitative PCR, a nucleic acid probe, which has a fluorescent substance and a quencher thereof, for example, at the 5' end and the 3' end, is hybridized with a predetermined nucleic acid. When the predetermined nucleic acid is amplified with DNA polymerase, the nucleic acid probe is decomposed by the exonuclease activity of the DNA polymerase, with the result that the fluorescent substance dissociates from the quencher and emits fluorescence. In this manner, the amount of the predetermined nucleic acid can be quantified (5'-nuclease method). Alternatively, the nucleic acid probe may be a single stranded chimeric oligonucleotide formed by connecting DNA, RNA and DNA sequentially in this order and may have, for example, a fluorescent substance and a quencher thereof at the 5' end and the 3' end. The single stranded chimeric oligonucleotide can be used in a cycling probe detection method.

In the specification, the "primer set" refers to a combination of a forward primer and a reverse primer that can be used for amplifying a predetermined sequence by a polymerase chain reaction (PCR). A primer is usually a single-stranded oligonucleotide having about a 20-30 mers in length. The primer sets for a plurality of genes refer to a combination of primer sets for individual genes. For example, the primer sets for two genes A and B means a combination of a primer set for gene A and a primer set for gene B.

In the specification, the "blocking the binding" refers to intercepting, blocking or reducing the interaction between two molecules. Accordingly, blocking the binding between PD-1 and a ligand thereof means intercepting, blocking or reducing the interaction between PD-1 and the ligand thereof. The binding can be blocked by using, for example, a binding-blocking antibody (for example, anti PD-1 antibody and anti PD-L1 antibody). In the specification, "blocking" is used with the intention to not only 100% blocking but also, for example, 90% or more, 80% or more, 70% or more, 60% or more, or 50% or more blocking. The "inhibiting," "reducing" or "intercepting" the interaction between PD-1 and a ligand thereof is used with the intention to not only 100% inhibition, reduction or interception but also, for example, 90% or more, 80% or more, 70% or more, 60% or more, or 50% or more inhibition, reduction or interception.

According to the present invention, there is provided a method for predicting the sensitivity of a cancer or a tumor of a subject to a treatment with a PD-1 immune checkpoint inhibitor, comprising measuring the expression level(s) of one or more genes selected from the group consisting of the following genes: ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B, GBP1, FAM211B, PRR5, C17orf67, FAM101B and TBL1X in a cancer sample or a tumor sample obtained from the subject.

In the specification, the phrase "method for predicting" can be replaced with the phrases "method for estimating," "assistive method for estimating," "an assistive method for predicting," "method for determining" or "assistive method for determining."

According to the present invention, for example, there is provided a method for predicting, estimating or determining the sensitivity of a cancer or a tumor of a subject to a treatment with a PD-1 immune checkpoint inhibitor or a assistive method for estimating, predicting or determining the sensitivity,

comprising measuring the expression level(s) of one or more genes selected from the group consisting of the following genes:

ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B, GBP1, FAM211B, PRR5, C17orf67, FAM101B and TBL1X in a cancer sample or a tumor sample obtained from the subject.

In the present invention, the treatment with a PD-1 immune checkpoint inhibitor may be a treatment for inhibiting suppression of function of PD-1 expressing T cells caused by binding between PD-1 and a ligand of PD-1 expressed on cells of some types of cancers and tumors, and preferably, a treatment for blocking, reducing, intercepting or inhibiting the binding between PD-1 and a ligand of PD-1 expressed on cells of some types of cancers and tumors. It is possible for those skilled in the art to obtain an anti PD-1 antibody or an anti PD-L1 antibody that blocks, reduces, intercepts or inhibits the binding between PD-1 and a ligand thereof by an antibody preparation technique and binding blocking assay. Besides antibodies, many PD-1 immune checkpoint inhibitors are known, the sensitivity can be evaluated in the present invention. The treatment for blocking, reducing, intercepting or inhibiting the binding between PD-1 and a ligand thereof can be performed by administering a PD-1 immune checkpoint inhibitor, for example, an anti PD-1 antibody or an anti PD-L1 antibody that reduces, intercepts or inhibits the binding between PD-1 and a ligand thereof, to a patient having a cancer or a tumor. The anti PD-1 antibody or the anti PD-L1 antibody contained in an anti-cancer agent is usually an anti PD-1 antibody or an anti PD-L1 antibody that reduces, intercepts or inhibits the binding between PD-1 and a ligand thereof. The anti PD-1 antibody or the anti PD-L1 antibody contained in an anti-cancer agent can be, more specifically, an anti PD-1 antibody or an anti PD-L1 antibody serving as an immune checkpoint inhibitor.

The PD-1 immune checkpoint inhibitor, the sensitivity to which is to be evaluated by the present invention, is not particularly limited if it is an immune checkpoint inhibitor targeting PD-1 or PD-L1; and can be, for example, immune checkpoint inhibitors shown in the following Table 1. The PD-1 immune checkpoint inhibitor, the sensitivity to which is to be evaluated by the present invention is not limited to an antibody and may be a low molecular-weight compound or cells as long as they inhibit suppressive action to T cells caused by the binding between PD-1 and a ligand thereof and serve as an immune checkpoint inhibitor.

[Table 1]

Table 1: Examples of immune checkpoint inhibitor targeting PD-1 or PD-L1

| Active ingredient | Name of immune checkpoint inhibitor |
|---|---|
| Anti PD-1 antibody | Pembrolizumab |
| | Nivolumab |
| | MK-3475 |
| | AMP-224 |
| | Pidilizumab (CT-01 1) |
| | AMP-514, MEDI-0680 |
| | ANB-011, TSR-042 |
| | BGB-A317, hu317-1/IgG4mt2 |
| | REGN-2810, SAR-439684 |
| | HR-301210, SHR-1210 |
| | PF-06801591 |
| | JS-001 |
| | IBI-308 |
| | CBT-501 |
| Anti PD-L1 antibody | Atezolizumab (MPDL3280A, RG7446) |
| | BMS-936559, MDX-1105 |
| | Durvalumab (MEDI-4736) |
| | Avelumab (MSB0010718C) |
| | LY-3300054 |
| | JNJ-61610588 |
| Pluripotent Killer T Cells Expressing Antibodies for PD-1 | PIK-PD-1 cells |
| PD-L1 inhibitor | AUPM-170, CA-170 (Oral, Small Molecule PD-L1/VISTA Antagonist) |

In the present invention, the subject that can be subjected to prediction by the present invention includes, for example, a subject having a cancer or a tumor, a subject diagnosed as having a cancer or a tumor, or a subject possibly having a cancer or a tumor. In the present invention, the subject that can be subjected to prediction by the present invention includes, for example, subjects before a treatment with a PD-1 immune checkpoint point inhibitor.

In the present invention, examples of the cancer or the tumor include, but are not particularly limited to, blood cancer, brain tumor, head and neck cancer, esophagus cancer, stomach cancer, appendicular cancer, colorectal cancer, anal cancer, gall bladder cancer, bile duct cancer, pancreatic cancer, gastrointestinal stromal tumor, lung cancer, liver cancer, mesothelioma, thyroid cancer, kidney cancer, prostate cancer, neuroendocrine tumor, malignant melanoma, breast cancer, uterine carcinoma, cervical cancer, ovarian cancer, osteosarcoma, soft tissue sarcoma, Kaposi's sarcoma, myosarcoma, kidney cancer, bladder cancer and testicular cancer. Of these, the examples include cancers which were confirmed to have the sensitivity to a treatment with a PD-1 immune checkpoint inhibitor; particularly, malignant melanoma, non-small cell lung cancer and renal cell cancer are preferable.

In the present invention, the sample is a sample obtained from a subject. The sample can be, for example, a sample obtained from a subject having a cancer or a tumor, or a subject diagnosed as having a cancer or a tumor. In this case, examples of the sample include, but are not limited to, tumor tissues obtained by biopsy, spinal fluid, intrathoracic fluid, intraperitoneal fluid, lymph fluid, skin section, blood, urine, feces, sputum, respiratory organ, intestinal tract, genitourinary tract, saliva, milk, digestive organ, and cells collected from these. Preferable examples can be a biopsy tissue sample of a cancer or a tumor, or a blood sample (for example, isolated, purified or concentrated whole blood sample or T cell sample).

In an embodiment of the present invention, in order to predict the sensitivity of a cancer or a tumor of a subject to a treatment with a PD-1 immune checkpoint inhibitor, the expression level of any one gene selected from the group consisting of the following genes: ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B, GBP1, FAM211B, PRR5, C17orf67, FAM101B and TBL1X is measured. In a specific case of the embodiment, the expression level of a gene in a biopsy tissue sample of a cancer or a tumor, or a biopsy tissue sample of a tissue suspected as being a cancer or a tumor is measured.

The expression level of a gene can be measured by using for example, a biopsy tissue sample of a cancer or a tumor. The expression level of a gene can be measured by using, for example, a biopsy tissue sample of a tissue suspected as being a cancer or a tumor.

In an embodiment of the present invention, in order to predict the sensitivity of a cancer or a tumor of a subject to a treatment with a PD-1 immune checkpoint inhibitor, the expression level(s) of any one or more genes (for example, the expression levels of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 or 36 genes) selected from the group consisting of the following 36 genes: ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B, GBP1, FAM211B, PRR5, C17orf67, FAM101B and TBL1X are measured. In a specific embodiment, the expression levels of all 36 genes mentioned above are measured.

In an embodiment of the present invention, in order to predict the sensitivity of a cancer or a tumor of a subject to a treatment with a PD-1 immune checkpoint inhibitor, the expression level(s) of one or more genes (for example, 2, 3, 4, 5, 6, 7, 8, 9, 10, 11, 12, 13, 14, 15, 16, 17, 18, 19, 20, 21, 22, 23, 24, 25, 26, 27, 28, 29, 30 or 31 genes) selected from the group consisting of the following 31 genes: ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B and GBP1 are measured. In a specific embodiment, the expression levels of all 31 genes are measured.

In an embodiment of the present invention, in order to predict the sensitivity of a cancer or a tumor of a subject to a treatment with a PD-1 immune checkpoint inhibitor, the expression level of any one gene selected from the group consisting of the following 31 genes: ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B and GBP1 is measured. In a specific case of the embodiment, the expression level of a gene in a biopsy tissue sample of a cancer or a tumor of a subject, or a biopsy tissue sample of a tissue suspected as being a cancer or a tumor is measured.

In an embodiment of the present invention, in order to predict the sensitivity of a cancer or a tumor of a subject to a treatment with a PD-1 immune checkpoint inhibitor, the expression level of a TBL1X gene is measured.

In an embodiment of the present invention, in order to predict the sensitivity of a cancer or a tumor of a subject to a treatment with a PD-1 immune checkpoint inhibitor, the expression level of an FAM101B gene is measured.

In an embodiment of the present invention, in order to predict the sensitivity of a cancer or a tumor of a subject to a treatment with a PD-1 immune checkpoint inhibitor, the expression level of a TBC1D4 gene is measured.

In an embodiment of the present invention, in order to predict the sensitivity of a cancer or a tumor of a subject to a treatment with a PD-1 immune checkpoint inhibitor, the expression level of a PRR5 gene is measured.

In an embodiment of the present invention, in order to predict the sensitivity of a cancer or a tumor of a subject to a treatment with a PD-1 immune checkpoint inhibitor, the expression level of a PTGDS gene is measured.

In an embodiment of the present invention, in order to predict the sensitivity of a cancer or a tumor of a subject to a treatment with a PD-1 immune checkpoint inhibitor, the expression level of a FAM211B gene is measured.

In an embodiment of the present invention, in order to predict the sensitivity of a cancer or a tumor of a subject to a treatment with a PD-1 immune checkpoint inhibitor, the expression level of a C17orf67 gene is measured.

In an embodiment of the present invention, in order to predict the sensitivity of a cancer or a tumor of a subject to a treatment with a PD-1 immune checkpoint inhibitor, the expression level of an RAB11FIP5 gene is measured.

In an embodiment of the present invention, in order to predict the sensitivity of a cancer or a tumor of a subject to a treatment with a PD-1 immune checkpoint inhibitor, the expression level of a GCNT2 gene is measured.

In an embodiment of the present invention, in order to predict the sensitivity of a cancer or a tumor of a subject to a treatment with a PD-1 immune checkpoint inhibitor, the expression level of a TSPAN32 gene is measured.

In an embodiment of the present invention, in order to predict the sensitivity of a cancer or a tumor of a subject to a treatment with a PD-1 immune checkpoint inhibitor, the expression level of an MYB gene is measured.

In an embodiment of the present invention, in order to predict the sensitivity of a cancer or a tumor of a subject to a treatment with a PD-1 immune checkpoint inhibitor, the expression level of an ADAM8 gene is measured.

In an embodiment of the present invention, in order to predict the sensitivity of a cancer or a tumor of a subject to a treatment with a PD-1 immune checkpoint inhibitor, the expression level of an ASAP1 gene is measured.

In an embodiment of the present invention, in order to predict the sensitivity of a cancer or a tumor of a subject to a treatment with a PD-1 immune checkpoint inhibitor, the expression level of an ITGB1 gene is measured.

In an embodiment of the present invention, in order to predict the sensitivity of a cancer or a tumor of a subject to a treatment with a PD-1 immune checkpoint inhibitor, the expression level of an MBOAT1 gene is measured.

In an embodiment of the present invention, in order to predict the sensitivity of a cancer or a tumor of a subject to a treatment with a PD-1 immune checkpoint inhibitor, the expression level of an ASB2 gene is measured.

In an embodiment of the present invention, in order to predict the sensitivity of a cancer or a tumor of a subject to a treatment with a PD-1 immune checkpoint inhibitor, the expression level of a COTL1 gene is measured.

In an embodiment of the present invention, in order to predict the sensitivity of a cancer or a tumor of a subject to a treatment with a PD-1 immune checkpoint inhibitor, the expression level of an FAM65B gene is measured.

In an embodiment of the present invention, in order to predict the sensitivity of a cancer or a tumor of a subject to a treatment with a PD-1 immune checkpoint inhibitor, the expression level of an SGPP2 gene is measured.

In an embodiment of the present invention, in order to predict the sensitivity of a cancer or a tumor of a subject to a treatment with a PD-1 immune checkpoint inhibitor, the expression level of an SLC4A8 gene is measured.

In an embodiment of the present invention, in order to predict the sensitivity of a cancer or a tumor of a subject to a treatment with a PD-1 immune checkpoint inhibitor, the expression level of an LEF1-AS1 gene is measured.

In an embodiment of the present invention, in order to predict the sensitivity of a cancer or a tumor of a subject to a treatment with a PD-1 immune checkpoint inhibitor, the expression level of an RXRA gene is measured.

In an embodiment of the present invention, in order to predict the sensitivity of a cancer or a tumor of a subject to a treatment with a PD-1 immune checkpoint inhibitor, the expression level of an NIN gene is measured.

In an embodiment of the present invention, in order to predict the sensitivity of a cancer or a tumor of a subject to a treatment with a PD-1 immune checkpoint inhibitor, the expression level of a VCL gene is measured.

In an embodiment of the present invention, in order to predict the sensitivity of a cancer or a tumor of a subject to a treatment with a PD-1 immune checkpoint inhibitor, the expression level of a KLRD1 gene is measured.

In an embodiment of the present invention, in order to predict the sensitivity of a cancer or a tumor of a subject to a treatment with a PD-1 immune checkpoint inhibitor, the expression level of an EMP3 gene is measured.

In an embodiment of the present invention, in order to predict the sensitivity of a cancer or a tumor of a subject to a treatment with a PD-1 immune checkpoint inhibitor, the expression level of a PTPN13 gene is measured.

In an embodiment of the present invention, in order to predict the sensitivity of a cancer or a tumor of a subject to a treatment with a PD-1 immune checkpoint inhibitor, the expression level of an AMPD3 gene is measured.

In an embodiment of the present invention, in order to predict the sensitivity of a cancer or a tumor of a subject to a treatment with a PD-1 immune checkpoint inhibitor, the expression level of an ICOS gene is measured.

In an embodiment of the present invention, in order to predict the sensitivity of a cancer or a tumor of a subject to a treatment with a PD-1 immune checkpoint inhibitor, the expression level of a PASK gene is measured.

In an embodiment of the present invention, in order to predict the sensitivity of a cancer or a tumor of a subject to a treatment with a PD-1 immune checkpoint inhibitor, the expression level of a CTBP2 gene is measured.

In an embodiment of the present invention, in order to predict the sensitivity of a cancer or a tumor of a subject to a treatment with a PD-1 immune checkpoint inhibitor, the expression level of an HNRPLL gene is measured.

In an embodiment of the present invention, in order to predict the sensitivity of a cancer or a tumor of a subject to a treatment with a PD-1 immune checkpoint inhibitor, the expression level of a PDCD1 gene is measured.

In an embodiment of the present invention, in order to predict the sensitivity of a cancer or a tumor of a subject to a treatment with a PD-1 immune checkpoint inhibitor, the expression level of an SIRPG gene is measured.

In an embodiment of the present invention, in order to predict the sensitivity of a cancer or a tumor of a subject to a treatment with a PD-1 immune checkpoint inhibitor, the expression level of a GBP1 gene is measured.

In an embodiment of the present invention, in order to predict the sensitivity of a cancer or a tumor of a subject to a treatment with a PD-1 immune checkpoint inhibitor, the expression level of an ST8SIA1 gene is measured.

In the above embodiments, if the expression level of a gene increases, in order to predict whether or not a cancer or a tumor of a subject shows "complete response" (CR) to a treatment with a PD-1 immune checkpoint inhibitor, for example, TBC1D4 can be preferably used.

In the above embodiments, if the expression level of a gene decreases, in order to predict whether or not a cancer or a tumor of a subject shows "complete response" (CR) to a treatment with a PD-1 immune checkpoint inhibitor, for example, TBL1X, FAM101B, PTGDS, PRR5, FAM211B, RAB11FIP5 or ASB2 can be preferably used.

In the above embodiments, if the expression level of a gene increases, in order to predict whether or not a cancer or a tumor of a subject shows "progressive disease" (PD) to a treatment with a PD-1 immune checkpoint inhibitor, for example, FAM101B, PRR5, PTGDS, FAM211B, RAB11FIP5, GCNT2, ITGB1 or MBOAT1 can be preferably used.

In the above embodiments, in order to predict the sensitivity of a cancer or a tumor of a subject to a treatment with a PD-1 immune checkpoint inhibitor, the expression level of one gene specified in the above embodiments, and further, the expression level(s) of one or more genes excluding the gene specified above and selected from the group consisting of the following 36 genes: ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B, GBP1, FAM211B, PRR5, C17orf67, FAM101B and TBL1X may be measured.

In an embodiment of the present invention, a gene signature can be any one of the combinations described in the following Table 2, for example.

[Table 2]

| Combination No. | Combination | | |
|---|---|---|---|
| 1 | TBL1X | FAM101B | |
| 2 | TBL1X | TBC1D4 | |
| 3 | TBL1X | PRR5 | |
| 4 | TBL1X | PTGDS | |
| 5 | FAM101B | TBC1D4 | |
| 6 | FAM101B | PRR5 | |
| 7 | FAM101B | PTGDS | |
| 8 | TBC1D4 | PRR5 | |
| 9 | TBC1D4 | PTGDS | |
| 10 | PRR5 | PTGDS | |
| 11 | TBL1X | FAM101B | TBC1D4 |
| 12 | TBL1X | FAM101B | PRR5 |
| 13 | TBL1X | FAM101B | PTGDS |
| 14 | TBL1X | TBC1D4 | PRR5 |
| 15 | TBL1X | TBC1D4 | PTGDS |
| 16 | TBL1X | PRR5 | PTGDS |
| 17 | FAM101B | TBC1D4 | PRR5 |
| 18 | FAM101B | TBC1D4 | PTGDS |
| 19 | FAM101B | PRR5 | PTGDS |
| 20 | TBC1D4 | PRR5 | PTGDS |

In the embodiments described in Table 2, in order to predict the sensitivity of a cancer or a tumor of a subject to a treatment with a PD-1 immune checkpoint inhibitor, expression levels of individual genes of a gene signature containing any one of the combinations are measured.

In an embodiment of the present invention, the gene signature contains TBL1X and FAM101B. In this embodiment, in order to predict the sensitivity of a cancer or a tumor of a subject to a treatment with a PD-1 immune checkpoint inhibitor, the expression levels of individual genes of the gene signature containing TBL1X and FAM101B are measured.

In an embodiment of the present invention, the gene signature contains TBL1X, FAM101B and TBC1D4. In this embodiment, in order to predict the sensitivity of a cancer or a tumor of a subject to a treatment with a PD-1 immune checkpoint inhibitor, the expression levels of individual genes of the gene signature containing TBL1X, FAM101B and TBC1D4 are measured.

In an embodiment of the present invention, the gene signature contains TBL1X, FAM101B, TBC1D4 and PRR5. In this embodiment, in order to predict the sensitivity of a cancer or a tumor of a subject to a treatment with a PD-1 immune checkpoint inhibitor, the expression levels of individual genes of the gene signature containing TBL1X, FAM101B, TBC1D4 and PRR5 are measured.

In an embodiment of the present invention, the gene signature contains TBL1X, FAM101B, TBC1D4, PRR5 and PTGDS. In this embodiment, in order to predict the sensitivity of a cancer or a tumor of a subject to a treatment with a PD-1 immune checkpoint inhibitor, the expression levels of individual genes of the gene signature containing TBL1X, FAM101B, TBC1D4, PRR5 and PTGDS are measured.

In an embodiment of the present invention, the gene signature contains TBL1X, FAM101B, TBC1D4, PRR5, PTGDS and RAB11FIP5. In this embodiment, in order to predict the sensitivity of a cancer or a tumor of a subject to a treatment with a PD-1 immune checkpoint inhibitor, the expression levels of individual genes of the gene signature containing TBL1X, FAM101B, TBC1D4, PRR5, PTGDS and RAB11FIP5 are measured.

In an embodiment of the present invention, the gene signature contains TBL1X, FAM101B, TBC1D4, PRR5, PTGDS, RAB11FIP5 and GCNT2. In this embodiment, in order to predict the sensitivity of a cancer or a tumor of a subject to a treatment with a PD-1 immune checkpoint inhibitor, the expression levels of individual genes of the gene signature containing TBL1X, FAM101B, TBC1D4, PRR5, PTGDS, RAB11FIP5 and GCNT2 are measured.

In an embodiment of the present invention, the gene signature contains TBL1X, FAM101B, TBC1D4, PRR5, PTGDS, RAB11FIP5, GCNT2 and FAM211B. In this embodiment, in order to predict the sensitivity of a cancer or a tumor of a subject to a treatment with a PD-1 immune checkpoint inhibitor, the expression levels of individual genes of the gene signature containing TBL1X, FAM101B, TBC1D4, PRR5, PTGDS, RAB11FIP5, GCNT2 and FAM211B are measured.

In an embodiment of the present invention, the gene signature contains TBL1X, FAM101B, TBC1D4, PRR5, PTGDS, RAB11FIP5, GCNT2, FAM211B and TSPAN32. In this embodiment, in order to predict the sensitivity of a cancer or a tumor of a subject to a treatment with a PD-1 immune checkpoint inhibitor, the expression levels of individual genes of the gene signature containing TBL1X, FAM101B, TBC1D4, PRR5, PTGDS, RAB11FIP5, GCNT2, FAM211B and TSPAN32 are measured.

In an embodiment of the present invention, the gene signature contains TBL1X, FAM101B, TBC1D4, PRR5, PTGDS, RAB11FIP5, GCNT2, FAM211B, TSPAN32 and MYB. In this embodiment, in order to predict the sensitivity of a cancer or a tumor of a subject to a treatment with a PD-1 immune checkpoint inhibitor, the expression levels of individual genes of the gene signature containing TBL1X, FAM101B, TBC1D4, PRR5, PTGDS, RAB11FIP5, GCNT2, FAM211B, TSPAN32 and MYB are measured.

In an embodiment of the present invention, in order to predict the sensitivity of a cancer or a tumor of a subject to a treatment with a PD-1 immune checkpoint inhibitor, the expression level(s) of 1, 2, 3, 4, 5, 6, 7, 8, 9 or 10 genes selected from the group consisting of TBL1X, FAM101B, TBC1D4, PRR5, PTGDS, RAB11FIP5, GCNT2, FAM211B, TSPAN32 and MYB are measured.

In an embodiment of the present invention, in order to predict the sensitivity of a cancer or a tumor of a subject to a treatment with a PD-1 immune checkpoint inhibitor, the expression level(s) of 1, 2, 3, 4, 5, 6, 7, 8 or 9 genes selected from the group consisting of TBL1X, FAM101B, TBC1D4, PRR5, PTGDS, RAB11FIP5, GCNT2, FAM211B and TSPAN32 are measured.

In an embodiment of the present invention, in order to predict the sensitivity of a cancer or a tumor of a subject to a treatment with a PD-1 immune checkpoint inhibitor, the expression level(s) of 1, 2, 3, 4, 5, 6, 7 or 8 genes selected from the group consisting of TBL1X, FAM101B, TBC1D4, PRR5, PTGDS, RAB11FIP5, GCNT2 and FAM211B are measured.

In an embodiment of the present invention, in order to predict the sensitivity of a cancer or a tumor of a subject to a treatment with a PD-1 immune checkpoint inhibitor, the expression level(s) of 1, 2, 3, 4, 5, 6 or 7 genes selected from the group consisting of TBL1X, FAM101B, TBC1D4, PRR5, PTGDS, RAB11FIP5 and GCNT2 are measured.

In an embodiment of the present invention, in order to predict the sensitivity of a cancer or a tumor of a subject to a treatment with a PD-1 immune checkpoint inhibitor, the expression level(s) of 1, 2, 3, 4, 5 or 6 genes selected from the group consisting of TBL1X, FAM101B, TBC1D4, PRR5, PTGDS and RAB11FIP5 are measured.

In an embodiment of the present invention, in order to predict the sensitivity of a cancer or a tumor of a subject to a treatment with a PD-1 immune checkpoint inhibitor, the expression level(s) of 1, 2, 3, 4 or 5 genes selected from the group consisting of TBL1X, FAM101B, TBC1D4, PRR5 and PTGDS are measured.

In an embodiment of the present invention, in order to predict the sensitivity of a cancer or a tumor of a subject to a treatment with a PD-1 immune checkpoint inhibitor, the expression level(s) of 1, 2, 3 or 4 genes selected from the group consisting of TBL1X, FAM101B, TBC1D4 and PRR5 are measured.

In an embodiment of the present invention, in order to predict the sensitivity of a cancer or a tumor of a subject to a treatment with a PD-1 immune checkpoint inhibitor, the expression level(s) of 1, 2 or 3 genes selected from the group consisting of TBL1X, FAM101B and TBC1D4 are measured.

In a specific embodiment of the present invention, the gene signature contains TBL1X and TBC1D4. In order to predict the sensitivity of a cancer or a tumor of a subject to a treatment with a PD-1 immune checkpoint inhibitor, the expression levels of individual TBL1X and TBC1D4 genes are measured.

In a specific embodiment of the present invention, the gene signature contains FAM101B and TBC1D4. In order to predict the sensitivity of a cancer or a tumor of a subject to a treatment with a PD-1 immune checkpoint inhibitor, the expression levels of individual FAM101B and TBC1D4 genes are measured.

In an embodiment of the present invention, the gene signature contains FAM211B, PRR5, C17orf67, FAM101B and TBL1X. In this embodiment, in order to predict the sensitivity of a cancer or a tumor of a subject to a treatment with a PD-1 immune checkpoint inhibitor, the expression levels of individual FAM211B, PRR5, C17orf67, FAM101B and TBL1X genes are measured.

In an embodiment of the present invention, the gene signature contains PTGDS, TBC1D4, FAM211B, PRR5, C17orf67, FAM101B and TBL1X. In this embodiment, in order to predict the sensitivity of a cancer or a tumor of a subject to a treatment with a PD-1 immune checkpoint inhibitor, the expression levels of individual PTGDS, TBC1D4, FAM211B, PRR5, C17orf67, FAM101B and TBL1X genes are measured.

In an embodiment of the present invention, in order to predict the sensitivity of a cancer or a tumor of a subject to a treatment with a PD-1 immune checkpoint inhibitor, the expression level of a PDCD1 gene is measured using a biopsy tissue sample of a cancer or a tumor. The expression level of a PDCD1 gene in a biopsy tissue sample is measured without isolating T cells invading into a cancer or a tumor.

The genes mentioned above have the following alias names.

[Table 3]

Table 3: Examples of genes constituting gene signatures

| Gene name | Alias name | EntrezGene ID |
|---|---|---|
| ADAM8 | A disintegrin and metalloproteinase domain 8 | 101 |
| VCL | vinculin | 7414 |
| LEF1-AS1 | LEF1 antisense RNA 1 | 641518 |
| TSPAN32 | tetraspanin 32 | 10077 |
| CTBP2 | C-terminal binding protein 2 | 1488 |
| RXRA | retinoid X receptor, alpha | 6256 |
| PTGDS | prostaglandin D2 synthase, brain | 5730 |
| ITGB1 | integrin, beta 1 (fibronectin receptor, beta polypeptide, antigen CD29 includes MDF2, MSK12) | 3688 |
| KLRD1 | killer cell lectin-like receptor subfamily D, member 1 | 3824 |
| RAB11FIP5 | RAB11 family interacting protein 5 | 26056 |
| SLC4A8 | solute carrier family 4, sodium bicarbonate cotransporter, member 8 | 9498 |
| NIN | ninein (GSK3B interacting protein) | 51199 |
| GCNT2 | glucosaminyl (N-acetyl) transferase 2, I-branching enzyme (I blood group) (N-acetyllactosaminide beta-1,6-N-acetylglucosaminyl-transferase) | 2651 |
| ASB2 | ankyrin repeat and SOCS box containing protein 2 | 51676 |
| HNRPLL | heterogeneous nuclear ribonucleoprotein L-like | 92906 |
| AMPD3 | adenosine monophosphate deaminase 3 | 272 |
| SIRPG | signal-regulatory protein, gamma | 55423 |
| PTPN13 | protein tyrosine phosphatase, non-receptor type 13 (APO-1/CD95 (Fas)-associated phosphatase) | 5783 |
| MYB | v-myb myeloblastosis viral oncogene homolog | 4602 |
| ASAP1 | Arf GTPase-activating protein with SH3 domain, ankyrin repeat and PH domain 1 | 50807 |
| MBOAT1 | membrane bound O-acyltransferase domain containing 1 | 154141 |
| COTL1 | coactosin-like protein 1 | 23406 |
| ICOS | inducible T-cell co-stimulator | 29851 |
| ST8SIA1 | ST8 alpha-N-acetyl-neuraminide alpha-2,8-sialyltransferase 1 | 6489 |
| PASK | PAS domain containing serine/threonine kinase | 23178 |

(continued)

| Gene name | Alias name | EntrezGene ID |
|---|---|---|
| SGPP2 | sphingosine-1-phosphate phosphatase 2 | 130367 |
| PDCD1 | programmed cell death 1 | 5133 |
| TBC1D4 | TBC1 domain family, member 4 | 9882 |
| EMP3 | epithelial membrane protein 3 | 2014 |
| FAM65B | family with sequence similarity 65, member B (myogenesis-related and NCAM-associated protein homolog) | 9750 |
| GBP1 | guanylate binding protein 1, interferon-inducible, 67-KD | 2633 |
| FAM211B | family with sequence similarity 211, member B (leucine-rich repeat-containing protein 75B) | 388886 |
| PRR5 | proline rich 5 | 55615 |
| C17orf67 | uncharacterized protein C17orf67 (chromosome 17 open reading frame 67) | 339210 |
| FAM101B | family with sequence similarity 101, member B (filamin-interacting protein FAM101B) | 359845 |
| TBL1X | transducin beta-like 1X-linked | 6907 |

The expression level of a gene can be measured by measuring the amount of a transcriptional product of the gene or the protein encoded by the gene. The transcriptional product of a gene can be quantified by those skilled in the art based on a technology known in the art (for example, quantitative PCR, digital PCR, northern hybridization, in-situ hybridization, sequencing of all transcriptomes). Examples of a method for extracting RNA from a sample obtained from a subject include an extraction method using phenol and a chaotropic salt, such as an extraction method using a commercially available kit such as TRIzol (manufactured by Invitrogen Corporation) and ISOGEN (manufactured by Wako Pure Chemical Industries, Ltd.); and methods using other commercially available kits such as RNAPrep total RNA extraction kit (manufactured by Beckman Coulter, Inc.), RNeasy Mini (manufactured by QIAGEN K.K.) and RNA Extraction Kit (manufactured by Pharmacia Biotech). Examples of a reverse transcriptase for use in preparing cDNA from extracted RNA include, but are not particularly limited to, reverse transcriptases derived from retroviruses such as Rous associated virus (RAV) and Avian myeloblastosis virus (AMV), and reverse transcriptases derived from mouse retroviruses such as Moloney murine leukemia virus (MMLV). Then, for example, an oligonucleotide primer and an oligonucleotide probe are used for an amplification reaction and a hybridization reaction, respectively, and an amplification product and a hybrid product are detected. Examples of such a detection method that can be used include an RT-PCR method, a northern blotting method, a dot blot method, a DNA array method, an *in-situ* hybridization method, an RNase protection assay and an mRNA-seq. It is possible for those skilled in the art to design oligonucleotide primers and oligonucleotide probes suitable for individual detection methods as mentioned above based on the nucleotide sequence of cDNA by customary methods. The amount of protein can be measured (quantified) by those skilled in the art based on techniques known in the art (e.g., an immunohistochemistry (immunostaining) method, a western blotting method, an ELISA method, flow cytometry, imaging cytometry, radioimmunoassay, an immunoprecipitation method, an analytical method using an antibody array).

In an embodiment of the present invention, if the expression level(s) of one or more genes selected from the group of genes represented by DOWN in Table 5-2 are high, the sensitivity to a treatment with a PD-1 immune checkpoint inhibitor can be evaluated as being low. In the prediction method of the present invention, if the expression level(s) of one or more genes selected from the group of genes represented by UP in Table 5-2 are high, the sensitivity to a treatment with a PD-1 immune checkpoint inhibitor can be evaluated as being high.

In an embodiment of the present invention, if the expression level(s) of one or more genes selected from the group of genes represented by DOWN in Table 5-2 are low, the sensitivity to a treatment with a PD-1 immune checkpoint inhibitor can be evaluated as being high. In the prediction method of the present invention, if the expression level(s) of one or more genes selected from the group of genes represented by UP in Table 5-2 are low, the sensitivity to a treatment with a PD-1 immune checkpoint inhibitor can be evaluated as being low.

In an embodiment of the present invention, with an increase of the number of genes showing a high expression level in the genes selected from the group of genes represented by DOWN in Table 5-2, the sensitivity to a treatment with a PD-1 immune checkpoint inhibitor can be evaluated as being low. In an embodiment of the present invention, with an increase of the number of genes showing a high expression level in the genes selected from the group of genes represented by UP in Table 5-2, the sensitivity to a treatment with a PD-1 immune checkpoint inhibitor can be evaluated as being high.

In other words, in an embodiment of the present invention, with an increase of the ratio of genes showing a high expression level in the genes selected from the group of genes represented by DOWN in Table 5-2, the sensitivity to a treatment with a PD-1 immune checkpoint inhibitor can be evaluated as being low. In an embodiment of the present invention, with an increase of the ratio of genes showing a high expression level in the genes selected from the group of genes represented by UP in Table 5-2, the sensitivity to a treatment with a PD-1 immune checkpoint inhibitor can be evaluated as being high.

In an embodiment of the present invention, with a decrease of the number of genes showing a high expression level in the genes selected from the group of genes represented by DOWN in Table 5-2, the sensitivity to a treatment with a PD-1 immune checkpoint inhibitor can be evaluated as being high. In an embodiment of the present invention, with a decrease of the number of genes showing a high expression level in the genes selected from the group of genes represented by UP in Table 5-2, the sensitivity to a treatment with a PD-1 immune checkpoint inhibitor can be evaluated as being low.

In other words, in an embodiment of the present invention, with a decrease of the ratio of genes showing a high expression level in the genes selected from the group of genes represented by DOWN in Table 5-2, the sensitivity to a treatment with a PD-1 immune checkpoint inhibitor can be evaluated as being high. In an embodiment of the present invention, with a decrease of the ratio of genes showing a high expression level in the genes selected from the group of genes represented by UP in Table 5-2, the sensitivity to a treatment with a PD-1 immune checkpoint inhibitor can be evaluated as being low.

In an embodiment of the present invention, the expression levels of a plurality of genes may be standardized and added. For example, the expression levels of genes are standardized. Then, in the cases of the genes defined as DOWN in Table 5-2, the values obtained by standardization are each multiplied by a negative numerical value (for example, -1), and then, the products are added; whereas, in the cases of the genes defined as UP in Table 5-2, the values obtained by standardization (or products obtained by multiplying the values by an integer) are added. In this manner, a score (hereinafter referred to as "gene signature score" in some cases) may be obtained from the expression levels of a plurality of genes. In this case, as the gene signature score increases, the sensitivity to a treatment with a PD-1 immune checkpoint inhibitor can be evaluated as being high; whereas, as the gene signature score decreases, the sensitivity to a treatment with a PD-1 immune checkpoint inhibitor can be evaluated as being low.

In the prediction method of the present invention, standardization may be made by using Z-value. The Z-value refers to a value obtained by subtracting an average value from individual data values and dividing the obtained values by a standard deviation. By using Z-value, it becomes possible to equally evaluate differences of expression levels of genes. In this case, the sensitivity can be evaluated by comparing the gene signature score based on the Z-value to the score of a control.

In the prediction method of the present invention, the expression levels of individual genes may be standardized as the ratios to the expression level of a control gene. If this method is employed, it becomes possible to equally evaluate differences of expression levels of genes. In this case, a logarithm (for example, Log, for example $Log_2$) of the ratio may be used for sensitivity evaluation. The logarithm of the ratio is positive if the ratio is larger than 1 and negative if the ratio is less than 1, and thus, suitably used for comparison to a control.

In the prediction method of the present invention, the gene signature score may be obtained based on, for example, the following mathematical formula:

[Expression 1]

$$Strength = \frac{1}{N} \sum_{i=1}^{N} s_i \cdot \beta_i$$
(Formula 1)

wherein $\beta i$ represents an expression value (the expression value may be standardized) of "i"th gene contained in a gene signature or a $Log_2$ value of fold change (for example, expression in subject/expression in control); Si represents +1 value if the "i"th gene is defined as "UP" and -1 value if the "i"th gene is defined as "DOWN"; N represents the number of genes contained in the gene signature; and "Strength" is a gene signature score; or a mathematical formula mathematically equivalent to this mathematical formula, and used for evaluation. The "mathematically equivalent" refers to, for example, a manner for evaluating individual gene expression values or gene signature scores by standardizing (or normalizing) the values or scores such that the median value thereof becomes 0. In an embodiment of the present invention, $\beta i$ in the above formula can be a value obtained by subtracting, from the $Log_2$ value of the expression value of the "i"th gene contained in a gene signature in an analysis sample, the $Log_2$ value of a median gene expression value in samples.

In an embodiment of the present invention, if the gene signature score calculated based on the above formula is a predetermined value or more, it can be evaluated that a cancer or a tumor is sensitive to a treatment with a PD-1 immune checkpoint inhibitor. The predetermined value can be a numerical value of, for example, 0 or more, 0.1 or more, 0.2 or more, or 0.3 or more as a gene signature score obtained by the above Formula 1 where $\beta i$ is a value obtained by subtracting, from the $Log_2$ value of the expression value of each gene, the $Log_2$ value of a median gene expression value of the each gene in samples.

In an embodiment of the present invention, if the gene signature score calculated based on the above formula is less than a predetermined value, it can be evaluated that a cancer or a tumor is not sensitive (is resistant or tolerable) to a treatment with a PD-1 immune checkpoint inhibitor. The predetermined value can be a numerical value of, for example, 0.2 or less, 0.1 or less, 0 or less, -0.1 or less, -0.2 or less, -0.3 or less, or -0.4 or less, as a gene signature score obtained by the above Formula 1 where $\beta i$ is a value obtained by subtracting, from the $Log_2$ value of the expression value of each gene, the $Log_2$ value of a median gene expression value of the each gene in samples.

In an embodiment of the present invention, if a gene signature score is obtained by using a mathematical formula mathematically equivalent to the formula mentioned above, the above predetermined value can be converted so as to correspond to the equivalent expression and used for evaluation.

In the prediction method of the present invention, the sensitivity of a cancer to a treatment with a PD-1 immune checkpoint inhibitor can be evaluated and determined by comparing to the sensitivity of a proper control. The control is preferably another subject having the same type of cancer or tumor. The proper control can be, for example, a subject having sensitivity to a treatment with a PD-1 immune checkpoint inhibitor (for example, a subject showing a complete response to the above treatment) or a subject resistant to the above treatment (for example, a subject showing "progressive disease" to the above treatment).

Alternatively, in the prediction method of the present invention, the sensitivity can be evaluated based on comparison with a median value, an average value or a predetermined value in analysis samples; a median value, an average value or a predetermined value in samples taken from non-lesion sites; or a median value, an average value or a predetermined value in samples taken from a lesion site. Alternatively, a standard sample may be prepared in advance for measuring a gene expression level, and the ratio in expression level of a sample to the standard sample may be calculated to evaluate the sensitivity.

For a subject predicted as having high sensitivity to a treatment with a PD-1 immune checkpoint inhibitor by the prediction method of the present invention, a treatment with a PD-1 immune checkpoint inhibitor can be effective. For example, in an embodiment, a subject predicted as having high sensitivity to a treatment with a PD-1 immune checkpoint inhibitor by the prediction method of the present invention can show "complete response" (CR). In an embodiment, a subject predicted as having high sensitivity to a treatment with a PD-1 immune checkpoint inhibitor by the prediction method of the present invention can show "partial response" (PR). In an embodiment, a subject predicted as having high sensitivity to a treatment with a PD-1 immune checkpoint inhibitor by the prediction method of the present invention can show "stable disease." The present invention may further include, if desired, determining whether a subject predicted as having high sensitivity to a treatment with a PD-1 immune checkpoint inhibitor shows either one of "complete response," "partial response," "stable disease" and "progressive disease."

For a subject predicted as having high sensitivity to a treatment with a PD-1 immune checkpoint inhibitor by the prediction method of the present invention, a treatment with a PD-1 immune checkpoint inhibitor can be effective. Accordingly, in the present invention, there is provided a pharmaceutical composition comprising an anti PD-1 antibody or an anti PD-L1 antibody for use in treating or preventing a cancer or a tumor in a subject predicted as having sensitivity to a treatment with a PD-1 immune checkpoint inhibitor by the prediction method of the present invention. In the present invention, there is also provided use of an anti PD-1 antibody or an anti PD-L1 antibody for producing a medicament for use in treating or preventing a cancer or a tumor in a subject predicted as having sensitivity to a treatment with a PD-1 immune checkpoint inhibitor by the prediction method of the present invention.

In the present invention, there is provided a method for treating or preventing a cancer or a tumor in a subject in need thereof, comprising predicting the sensitivity of a cancer or a tumor of a subject to a treatment with a PD-1 immune checkpoint inhibitor by the prediction method of the present invention and applying a treatment with a PD-1 immune checkpoint inhibitor to a subject predicted as having the sensitivity.

In an embodiment of the present invention, there is provided a method for treating or preventing a cancer or a tumor in a subject in need thereof, comprising predicting the sensitivity of a cancer or a tumor of a subject to a treatment with a PD-1 immune checkpoint inhibitor by the prediction method of the present invention and administering an anti PD-1 antibody or an anti PD-L1 antibody to a subject predicted as having the sensitivity.

In another embodiment of the present invention, there is provided a gene signature for predicting the sensitivity to a treatment with a PD-1 immune checkpoint inhibitor.

The gene signature, in an embodiment, contains one or more genes (for example, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 or 36 genes) selected from the group consisting of ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B, GBP1, FAM211B, PRR5, C17orf67, FAM101B and TBL1X.

The gene signature, in an embodiment, contains one or more genes (for example, 2, 3, 4, 5, 6, 7, 8, 9, 10, 11, 12, 13, 14, 15, 16, 17, 18, 19, 20, 21, 22, 23, 24, 25, 26, 27, 28, 29, 30 or 31 genes) selected from the group consisting of ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B and GBP1.

In an embodiment of the present invention, there are provided a gene signature containing at least one or more genes selected from TBL1X, FAM101B, TBC1D4, PRR5 and PTGDS; and a gene signature containing all genes of at least TBL1X, PTGDS, FAM101B, FAM211B, TBC1D4, PRR5 and C17orf67.

In the present invention, there is provided a combination of nucleic acid probes or a combination of primer sets or a combination of nucleic acid probes and primer sets to a gene transcriptional product of each of the aforementioned genes or the genes contained in the gene signatures. In the present invention, there is provided an antibody or a combination of antibodies to a protein encoded by each of the aforementioned genes or the genes contained in the gene signatures. These combinations can be used for measuring the expression level of each of the aforementioned genes or the genes contained in the gene signatures. These combinations can be used for measuring the expression level of each of the aforementioned genes or the genes contained in the gene signatures in a biopsy tissue sample.

In the present invention, there is provided a composition comprising a combination of nucleic acid probes or a combination of primer sets or a combination of nucleic acid probes and primer sets of the present invention, for use in estimating the sensitivity to a treatment with a PD-1 immune checkpoint inhibitor. In the present invention, there is provided a composition comprising an antibody or a combination of antibodies to a protein encoded by each of the aforementioned genes or the genes contained in the gene signatures, for use in estimating the sensitivity to a treatment with a PD-1 immune checkpoint inhibitor. These compositions can be used for measuring the expression level of each of the aforementioned genes or the genes contained in the gene signatures. These compositions can be used for measuring the expression level of each of the aforementioned genes or the genes contained in the gene signatures, in a biopsy tissue sample.

In the present invention, there is also provided an array or a microarray for analyzing gene expression, having probes for a transcriptional product of each of the genes contained in the gene signatures fixed onto the surface thereof. The array or the microarray of the present invention has for example, 1000 or less, 900 or less, 800 or less, 700 or less, 600 or less, 500 or less, 400 or less, 300 or less, 200 or less, 100 or less, 90 or less, 80 or less, 70 or less, 60 or less, 50 or less, 45 or less, or 40 or less types of nucleic acid probes fixed onto the surface thereof. In a specific embodiment, the array or the microarray of the present invention has only probes to each of the genes contained in the gene signatures, which are target genes for evaluation of expression, fixed onto the surface.

On the array or the microarray, for example, probes to transcriptional products of individual genes contained in a gene signature are spotted. If a transcriptional product of each of the genes contained in the gene signature comes to be contact with a spot, each the genes can be hybridized with the probe on the spot. The hybridized transcriptional product of each gene can be detected, for example, by a label (for example, fluorescence, radioisotope, enzyme) attached to the transcriptional product of each gene in accordance with a customary method. The transcriptional product of each of the genes contained in a gene signature can be previously subjected to a treatment, for example, extraction from a sample and preparation of labeled RNA. These treatments can be carried out in accordance with methods known to those skilled in the art.

In the present invention, there is provided a kit for use in estimating the sensitivity to a treatment with a PD-1 immune checkpoint inhibitor, comprising means for measuring expression levels of a plurality of genes (for example, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 or 36 genes) selected from the group consisting of
ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B, GBP1, FAM211B, PRR5, C17orf67, FAM101B and TBL1X.

In the present invention, there is also provided a kit for use in estimating the sensitivity to a treatment with a PD-1 immune checkpoint inhibitor, comprising means for measuring expression levels of a plurality of genes (for example, 2, 3, 4, 5, 6, 7, 8, 9, 10, 11, 12, 13, 14, 15, 16, 17, 18, 19, 20, 21, 22, 23, 24, 25, 26, 27, 28, 29, 30 or 31 genes) selected from the group consisting of ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B and GBP1.

The kit of the present invention can be used for, for example, a subject having a cancer or a tumor, a subject diagnosed as having a cancer or a tumor, or a subject having a possibility of having a cancer or a tumor.

Means for measuring an expression level may include a nucleic acid probe and/or a primer set. Means for measuring an expression level may include an array or a microarray containing a nucleic acid probe.

Means for measuring an expression level may include an antibody that binds to a protein encoded by each gene. In this case, a labeled secondary antibody may be contained as measuring means. Means for measuring an expression level may include an ELISA measuring kit containing an antibody that binds to a protein encoded by each gene may be used.

In an embodiment of the present invention, there is provided a kit for use in estimating the sensitivity to a treatment with a PD-1 immune checkpoint inhibitor in a subject having a cancer or a tumor, a subject diagnosed as having a cancer or a tumor, or a subject having a possibility of having a cancer or a tumor, by measuring the expression of the following genes in a biopsy tissue sample of a cancer or a tumor, or a biopsy tissue sample of a tissue suspected as being a cancer or a tumor, comprising means for measuring the expression level(s) of one or more genes (for example, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 or 36 genes) selected from the group consisting of the following genes: ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B, GBP1, FAM211B, PRR5, C17orf67, FAM101B and TBL1X.

In the present invention, there is also provided a kit for use in estimating the sensitivity to a treatment with a PD-1 immune checkpoint inhibitor in a subject having a cancer or a tumor, a subject diagnosed as having a cancer or a tumor, or a subject having a possibility of having a cancer or a tumor by measuring the expression of the following genes in a biopsy tissue sample of a cancer or a tumor, or a biopsy tissue sample of a tissue suspected as being a cancer or a tumor, comprising means for measuring the expression level(s) of one or more genes (for example, 2, 3, 4, 5, 6, 7, 8, 9, 10, 11, 12, 13, 14, 15, 16, 17, 18, 19, 20, 21, 22, 23, 24, 25, 26, 27, 28, 29, 30 or 31 genes) selected from the group consisting of the following genes: ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B and GBP1. There are further provided a kit comprising means for measuring the expression level(s) of one or more genes selected from at least TBL1X, FAM101B, TBC1D4, PRR5 and PTGDS, and a kit comprising means for measuring the expression levels of all of at least TBL1X, PTGDS, FAM101B, FAM211B, TBC1D4, PRR5 and C17orf67.

The kits may contain an instruction for estimating the sensitivity to a treatment with a PD-1 immune checkpoint inhibitor in a biopsy tissue sample of a cancer or a tumor, or a biopsy tissue sample of a tissue suspected as being a cancer or a tumor by measuring the expression of the following genes.

In an embodiment of the present invention, the method of the present invention can be carried out in order to specify a patient sensitive to a treatment with a PD-1 immune checkpoint inhibitor. In this embodiment, for example, a patient on whom a treatment with a PD-1 immune checkpoint inhibitor exerts an effect is selected and a drug is administered to the patient. This will be advantageous because unnecessary drug administration to a patient on whom the drug does not exert an effect can be avoided.

In an embodiment of the present invention, the method of the present invention can be carried out in order to specify a patient tolerant to a treatment with a PD-1 immune checkpoint inhibitor. In this embodiment, for example, a patient on whom a treatment with a PD-1 immune checkpoint inhibitor does not exert an effect is eliminated. This will be advantageous because unnecessary drug administration to a patient on whom the drug does not exert an effect can be avoided.

In an embodiment of the present invention, a drug may be administered or not administered to a patient predicted as having a medium degree of sensitivity or only a medium degree of sensitivity to a treatment with a PD-1 immune checkpoint inhibitor. A preferable one can be selected in consideration of e.g., cost-effectiveness and escalation of medical expense.

Examples

Example 1: Acquisition of marker gene for evaluating sensitivity to therapy with anti PD-1 drug

In this Example, a marker gene for evaluating the sensitivity to a therapy with an anti PD-1 drug (more specifically, the sensitivity of a cancer to a treatment with an anti PD-1 antibody serving as an immune checkpoint inhibitor) was obtained.

Gene expression data, which was analysis for expression of genes of PD-1 positive and negative T cells, were searched from the Gene expression omnibus (GEO; http://www.ncbi.nlm.nih.gov/gds) and three data sets were obtained. The data sets obtained were as shown in the following Table 4-1 (hereinafter, the data set obtained will be collectively referred to as "data set

A") .

[Table 4-1]

Table 4-1. Gene expression data (data set A) obtained

| Data set | GEO Accession No. | Title | Sample | Comparison of gene expression data |
|---|---|---|---|---|
| A | GSE26495 | Phenotype, Function and Gene Expression Profiles of PD-1 high CD8 T cells in Healthy Human Adults | CD8+ T cell | PD-1 high and PD-1 low |
| A | GSE50725 | Foxp1 is a rate-limiting and essential negative regulator of Tfh cell differentiation | T cell OVA specific Foxp1 | CXCR5 high PD-1 high and CXCR5 - PD-1- |
| A | GSE52561 | Gene expression profiling of CD4+ T-cell subsets in follicular lymphoma. | CD4+ T cell | PD-1 high CXCR5 high and PD-1 low CXCR5 low |

Gene expression data, which were evaluation of the sensitivity of a cancer to a treatment with an anti PD-1 antibody carried out in biopsy tissue samples taken from malignant melanoma patients before treatment, were obtained (see, Table 4-2; hereinafter, the data set obtained will be referred to as "data set B").

[Table 4-2]

Table 4-2. Gene expression data (date set B) obtained

| Data set | GEO Registration No. | Title | Sample | Comparison of gene expression data |
|---|---|---|---|---|
| B | GSE78220 | mRNA expressions in pre-treatment melanomas undergoing anti-PD-1 checkpoint inhibition therapy | Biopsy tissue sample of melanoma | Complete response and progressive disease |

Search of marker gene from data set A

From each of data set A, genes whose expression significantly changed in PD-1 positive T cells were searched (fold change > 1.2, P < 0.05). Genes which were confirmed to reproducibly change in expression in two or more out of three data sets were subjected to calculation of an average amount of expression change. The genes showing an average amount of expression change of 1.5 times or more were selected (the genes of Nos. 1 to 31 listed in Table 5-1). The fold changes (> 1.2, p < 0.05) of individual genes in each of the data sets were as shown in Table 5-1. Averages fold changes (> 1.5, number of data sets > 2) were as shown in Table 5-1.

Note that, in the table, "Entrez gene ID" is ID for use in identifying a gene in the database provided by the National Center for Biotechnology Information (NCBI) in the U.S.A. The detailed information of the gene can be obtained based on the ID.

[Table 5-1]

Table 5-1. Analysis results of gene expression

| No | Gene name | Entrez Gene ID | Fold Change (>1.2, P<0.05) | | | | Average fold change (> 1.5, number of data sets > 2) | | | |
| | | | Data set A | | | Data set B | Data set A | | Data set A + B | |
| | | | GSE26495 | GSE50725 | GSE52561 | GSE78220 | Average fold change | Number of data | Average fold change | Number of data |
|---|---|---|---|---|---|---|---|---|---|---|
| 1 | ADAM8 | 101 | -1.24 | | -10.1 | | -5.67 | 2 | -5.67 | 2 |
| 2 | VCL | 7414 | -1.21 | | -6.85 | | -4.03 | 2 | -4.03 | 2 |
| 3 | LEF1-AS1 | 641518 | -1.2 | | -3.92 | | -2.56 | 2 | -2.56 | 2 |
| 4 | TSPAN32 | 10077 | -1.35 | | -3.17 | | -2.26 | 2 | -2.26 | 2 |
| 5 | CTBP2 | 1488 | -2.62 | | -1.24 | | -1.93 | 2 | -1.93 | 2 |
| 6 | RXRA | 6256 | -1.21 | | -2.57 | | -1.89 | 2 | -1.89 | 2 |
| 7 | PTGDS | 5730 | -1.75 | | -1.84 | | -1.80 | 2 | -1.80 | 2 |
| 8 | ITGB1 | 3688 | -1.43 | -1.98 | -1.94 | | -1.78 | 3 | -1.78 | 3 |
| 9 | KLRD1 | 3824 | -2.03 | -1.34 | | | -1.69 | 2 | -1.69 | 2 |
| 10 | RAB11FIP5 | 26056 | -1.6 | -1.76 | | | -1.68 | 2 | -1.68 | 2 |
| 11 | SLC4A8 | 9498 | -1.31 | | -1.71 | | -1.51 | 2 | -1.51 | 2 |
| 12 | NIN | 51199 | 1.25 | | 1.75 | | 1.50 | 2 | 1.50 | 2 |
| 13 | GCNT2 | 2651 | 1.54 | | 1.47 | | 1.51 | 2 | 1.51 | 2 |
| 14 | ASB2 | 51676 | 1.58 | | 1.65 | | 1.62 | 2 | 1.62 | 2 |
| 15 | HNRPLL | 92906 | 1.97 | | 1.56 | | 1.77 | 2 | 1.77 | 2 |
| 16 | AMPD3 | 272 | 1.59 | | 2.07 | | 1.83 | 2 | 1.83 | 2 |
| 17 | SIRPG | 55423 | 2.52 | | 1.25 | | 1.89 | 2 | 1.89 | 2 |
| 18 | PTPN13 | 5783 | 1.3 | | 3.23 | | 2.27 | 2 | 2.27 | 2 |
| 19 | MYB | 4602 | 1.28 | | 3.54 | | 2.41 | 2 | 2.41 | 2 |
| 20 | ASAP1 | 50807 | 1.41 | | 3,49 | | 2.45 | 2 | 2.45 | 2 |
| 21 | M80AT1 | 154141 | 1.75 | | 3.61 | | 2.68 | 2 | 2.68 | 2 |
| 22 | COTL1 | 23406 | 2.91 | | 2.87 | | 2.89 | 2 | 2.89 | 2 |
| 23 | ICOS | 29851 | 2.06 | | 3.95 | | 3.01 | 2 | 3.01 | 2 |
| 24 | ST8SIA1 | 6489 | 2.35 | | 4.96 | | 3.66 | 2 | 3.66 | 2 |
| 25 | PASK | 23178 | 2.12 | | 6.39 | | 4.26 | 2 | 4.26 | 2 |
| 26 | SGPP2 | 130367 | 1.88 | | 7,43 | | 4.66 | 2 | 4.66 | 2 |
| 27 | PDCD1 | 5133 | 1.35 | | 17.3 | | 9.33 | 2 | 9.33 | 2 |
| 28 | TBC1D4 | 9882 | 2.29 | | 2.23 | 4.30 | 2.26 | 2 | 2.94 | 3 |
| 29 | EMP3 | 2014 | | -1.5 | -13.2 | | -7.35 | 2 | -7.35 | 2 |

EP 3 546 591 A1

| No | Gene name | Entrez Gene ID | Fold Change (>1.2, P<0.05) | | | | Average fold change (> 1.5, number of data sets > 2) | | | |
| --- | --- | --- | --- | --- | --- | --- | --- | --- | --- | --- |
| | | | Data set A | | | Data set B | Data set A | | Data set A + B | |
| | | | GSE26495 | GSE50725 | GSE52561 | GSE78220 | Average fold change | Number of data | Average fold change | Number of data |
| 30 | FAM65B | 9750 | | -1.4 | -2.65 | | -2.03 | 2 | -2.03 | 2 |
| 31 | GBP1 | 2633 | | -1.75 | -2.03 | | -1.89 | 2 | -1.89 | 2 |
| 32 | FAM211B | 388886 | | -1.49 | | -4.90 | | | -3.20 | 2 |
| 33 | PRR5 | 55615 | | | -2.2 | -3.72 | | | -2.96 | 2 |
| 34 | C17orf67 | 339210 | | | -1.88 | -3.71 | | | -2.79 | 2 |
| 35 | FAM101B | 359845 | | | -2.36 | -3.10 | | | -2.73 | 2 |
| 36 | TBL1X | 6907 | | -127 | | -3.22 | | | -2.25 | 2 |

Based on the fold changes obtained, genes whose expression increases in the PD-1 positive samples are indicated by "UP" and genes whose expression reduces in the PD-1 positive samples are indicated by "DOWN," compared to that in the PD-1 negative samples, as summarized in Table 5-2 (see, data set A in Table 5-2).

[Table 5-2]

Table 5-2. Analysis results of gene expression

| | | Contents of gene set | | | | |
|---|---|---|---|---|---|---|
| | Gene set A | | | Gene set A+B | | |
| No | Gene name | EntrezGene ID | Up or down of expression | Gene name | EntrezGene ID | Up or down of expression |
| 1 | ADAM8 | 101 | DOWN | ADAM8 | 101 | DOWN |
| 2 | VCL | 7414 | DOWN | VCL | 7414 | DOWN |
| 3 | LEF1-AS1 | 641518 | DOWN | LEF1-AS1 | 641518 | DOWN |
| 4 | TSPAN32 | 10077 | DOWN | TSPAN32 | 10077 | DOWN |
| 5 | CTBP2 | 1488 | DOWN | CTBP2 | 1488 | DOWN |
| 6 | RXRA | 6256 | DOWN | RXRA | 6256 | DOWN |
| 7 | PTGDS | 5730 | DOWN | PTGDS | 5730 | DOWN |
| 8 | ITGB1 | 3688 | DOWN | ITGB1 | 3688 | DOWN |
| 9 | KLRD1 | 3824 | DOWN | KLRD1 | 3824 | DOWN |
| 10 | RAB11FIP5 | 26056 | DOWN | RAB11FIP5 | 26056 | DOWN |
| 11 | SLC4A8 | 9498 | DOWN | SLC4A8 | 9498 | DOWN |
| 12 | NIN | 51199 | UP | NIN | 51199 | UP |
| 13 | GCNT2 | 2651 | UP | GCNT2 | 2651 | UP |
| 14 | ASB2 | 51676 | UP | ASB2 | 51676 | UP |
| 15 | HNRPLL | 92906 | UP | HNRPLL | 92906 | UP |
| 16 | AMPD3 | 272 | UP | AMPD3 | 272 | UP |
| 17 | SIRPG | 55423 | UP | SIRPG | 55423 | UP |
| 18 | PTPN13 | 5783 | UP | PTPN13 | 5783 | UP |
| 19 | MYB | 4602 | UP | MYB | 4602 | UP |
| 20 | ASAP1 | 50807 | UP | ASAP1 | 50807 | UP |
| 21 | MBOAT1 | 154141 | UP | MBOAT1 | 154141 | UP |
| 22 | COTL1 | 23406 | UP | COTL1 | 23406 | UP |
| 23 | ICOS | 29851 | UP | ICOS | 29851 | UP |
| 24 | ST8SIA1 | 6489 | UP | ST8SIA1 | 6489 | UP |
| 25 | PASK | 23178 | UP | PASK | 23178 | UP |
| 26 | SGPP2 | 130367 | UP | SGPP2 | 130367 | UP |
| 27 | PDCD1 | 5133 | UP | PDCD1 | 5133 | UP |
| 28 | TBC1D4 | 9882 | UP | TBC1D4 | 9882 | UP |
| 29 | EMP3 | 2014 | DOWN | EMP3 | 2014 | DOWN |
| 30 | FAM65B | 9750 | DOWN | FAM65B | 9750 | DOWN |
| 31 | GBP1 | 2633 | DOWN | GBP1 | 2633 | DOWN |
| 32 | | | | FAM211B | 388886 | DOWN |
| 33 | | | | PRR5 | 55615 | DOWN |
| 34 | | | | C17orf67 | 339210 | DOWN |
| 35 | | | | FAM101B | 359845 | DOWN |
| 36 | | | | TBL1X | 6907 | DOWN |

Search of marker gene from data sets A and B

Next, genes confirmed to significantly change in expression between a "complete response" group and a "progressive disease" group were searched from data set B (fold change > 1.2, P < 0.05). Of the genes together with the genes of data set A, the genes confirmed to reproducibly change in expression were subjected to calculation of an average amount of expression change. The genes showing an average amount of expression change of 1.5 times or more were selected (see, "data set A + B" in Table 5-1 and "gene set A + B" in Table 5-2).

Of the genes selected by the aforementioned manner, a gene increased in the amount of gene expression change in "complete response" samples compared to a "progressive disease" sample was defined as "UP," and a gene decreased in the amount of gene expression change was defined as "DOWN." Gene set A was defined from expression data of genes derived from PD-1 positive T cells and gene set A + B was defined as gene set A + patient's data (see Table 5-2) .

Note that, gene set A consisted of 31 genes and gene set A + B consisted of 36 genes having 5 genes added.

Individual genes contained in gene sets shown in Table 5-1 and Table 5-2 are genes confirmed to reproducibly change in expression in different data sets. For the reason, the genes of Nos. 1 to 31 are useful in estimating whether a subject has PD-1 positive T cells. The genes of Nos. 1 to 31 and 32 to 36 are each useful in estimating whether a patient has a tumor satisfactorily responding to a treatment with a PD-1 immune checkpoint inhibitor.

Example 2: Preparation of gene signature and analysis of patient data

Gene signatures were prepared from the gene sets obtained in Example 1.

More specifically, gene signature A was prepared from all genes contained in gene set A and gene signature A + B was prepared from all genes contained in gene set A + B.

Gene signature scores were calculated by employing a scoring algorithm, i.e., Network Perturbation Amplitude (NPA) scoring algorithms based on reports by Martin et al. (BMC Systems Biology, 2012, 6:54). With respect to biopsy tissue samples of patients with malignant melanoma before treatment, an average expression value (Strength) of all genes defined in a gene signature was calculated as a gene signature score. A specific calculation expression is as follows:

[Expression 2]

$$Strength = \frac{1}{N} \sum_{i=1}^{N} s_i \cdot \beta_i$$

wherein $\beta i$ represents a value obtained by subtracting, from the $Log_2$ value of the expression value of the "i"th gene contained in a gene signature in an analysis sample, the $Log_2$ value of a median gene expression value in samples; Si represents +1 value if the "i"th gene is defined as "UP" and -1 value if the "i"th gene is defined as "DOWN"; and N represents the number of genes contained in the gene signature.

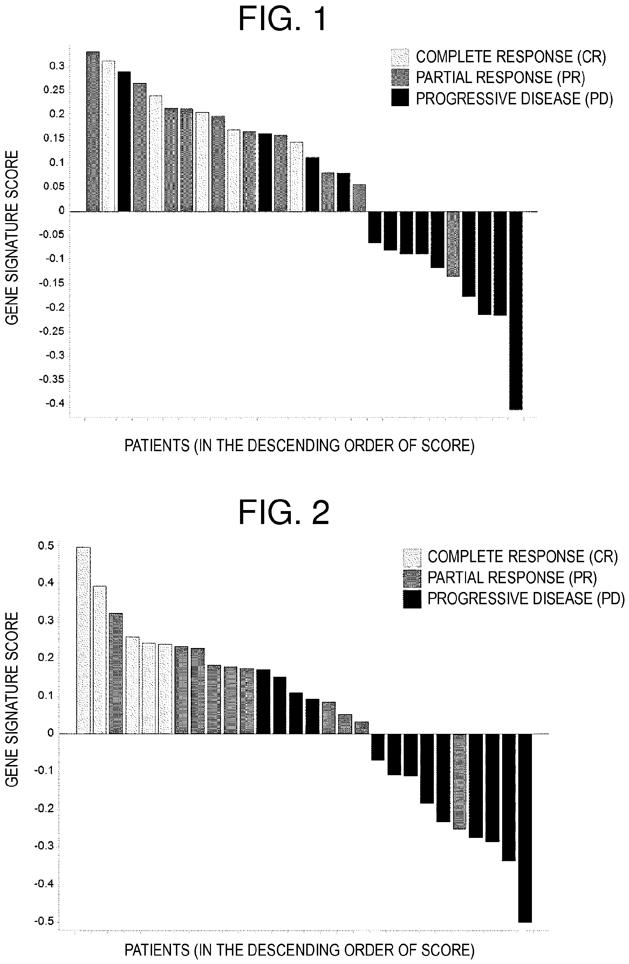

Next, from biopsy data of malignant melanoma, the gene signature score per patient was calculated. More specifically, using biopsy data (GSE78220) of malignant melanoma obtained from GEO, a gene signature score was calculated. The results were as shown in Figure 1 and Figure 2.

As shown in Figure 1, in patients of "progressive disease" having a low sensitivity to a treatment with an anti PD-1 antibody, the scores calculated from gene signature A were observed to tend to be low (for example, score was 0 or less).

As shown in Figure 2, in patients of "progressive disease" having a low sensitivity to a treatment with an anti PD-1 antibody, the scores calculated from gene signature A + B were observed to tend to be low (for example, score was 0 or less). In addition, as shown in Figure 2, in patients of "complete response" having a high sensitivity to a treatment with an anti PD-1 antibody, the scores calculated from gene signature A + B tended to be high.

Note that, in patients of "complete response," the score was 0 or more in all 5 cases; whereas in patients of "partial response," the score was 0 or more in 9 out of 10 cases. From this, it was found that the sensitivity of a cancer to a treatment with an anti PD-1 drug can be satisfactorily evaluated by a gene signature. In patients of "progressive disease," the score was less than 0 in 9 out of 13 cases. From this, it was found that resistance (tolerability) of a cancer to a treatment with an anti PD-1 drug can also be satisfactorily evaluated.

The gene signature scores of patients confirmed to have the sensitivity to a treatment with an anti PD-1 antibody were high in both gene signatures A and A + B.

Patients evaluated as having a score of 0 or more based on calculation from gene signature A were also evaluated as having a score of 0 or more based on calculation from gene signature A + B. Patients evaluated as having a score of less than 0 based on calculation from gene signature A were also evaluated as having a score of less than 0 based on calculation from gene signature A + B.

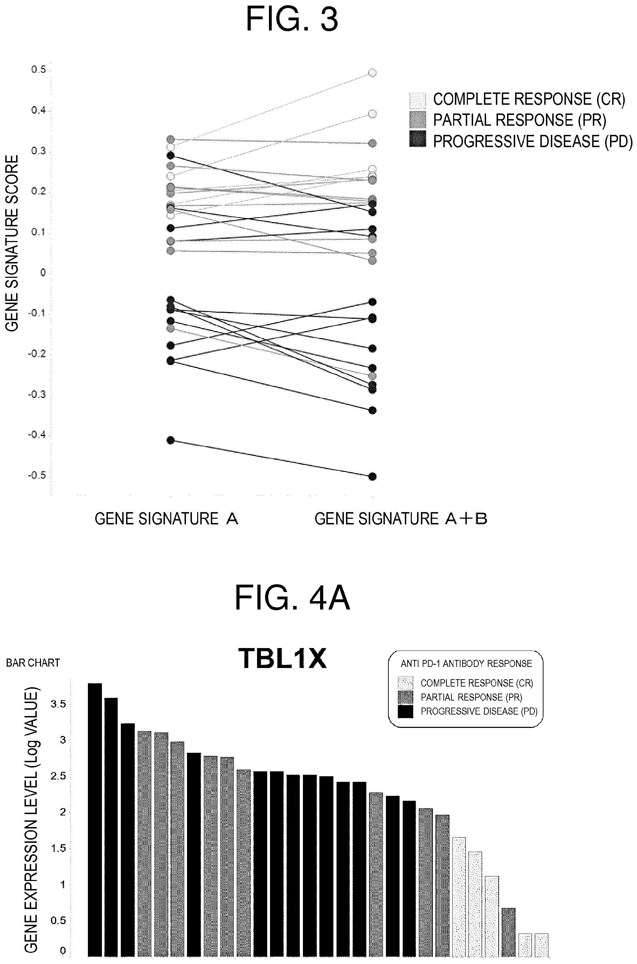

Whether the sensitivity of a cancer or a tumor to a treatment with a PD-1 immune checkpoint inhibitor can be predicted by a single gene was checked. The results were as shown in Table 6 and Figure 4A to Figure 4II. As shown in Table 6 and Figure 4A to Figure 4II, the expression level of each of the genes was correlated with the sensitivity to a treatment with a PD-1 immune checkpoint inhibitor. From this, it was apparent that the sensitivity of a cancer to a treatment with a PD-1 immune checkpoint inhibitor can be evaluated by using only one gene.

Note that Table 6 shows p value (reference value) obtained by t-test on the relationship between CR + PR vs PD and evaluation results based on gene expression values, and p value (reference value) obtained by t-test on the relationship between CR vs PD and evaluation results based on gene expression values. In the following table, a p value beyond 0.05 does not mean that prediction is impossible. For example, SLC4A8 shows p value of about 0.4; however, as shown in Figure 4S, if the threshold of a gene expression level is set to be, for example, about 0.1 to 0.5, a "partial response" or "progressive disease" case can be estimated with a prediction accuracy of 100%. If the threshold is set to be high, false positive and sensitivity decrease. In contrast, if the threshold is set to be low, false positive and sensitivity increase. The high and low of the threshold are directly correlated with prediction accuracy and prediction sensitivity. Thus, it is not correct that clinical significance of prediction is evaluated only based on high and low of p value.

[Table 6]

Table 6: Evaluation of response to immune checkpoint inhibitor by gene measurement

| Gene measured | p value by t-test (CR+PR vs PD) | p value by t-test t-test (CR vs PD) |
|---|---|---|
| TBL1X | 0.01746 | 0.00001 |
| FAM101B | 0.02894 | 0.0061 |
| TBC1D4 | 0.0324 | 0.00408 |
| PRR5 | 0.06209 | 0.01174 |
| PTGDS | 0.09223 | 0.02411 |
| FAM211B | 0.13417 | 0.03162 |
| C17orf67 | NA | NA |
| RAB11FIP5 | 0.12662 | 0.09111 |
| GCNT2 | 0.12958 | 0.20856 |
| TSPAN32 | 0.14072 | 0.77414 |
| MYB | 0.21311 | 0.55135 |
| ADAM8 | 0.25754 | 0.64437 |
| ASAP1 | 0.26841 | 0.77997 |
| ITGB1 | 0.30751 | 0.74304 |
| MBOAT1 | 0.3371 | 0.09636 |
| ASB2 | 0.34871 | 0.46099 |
| COTL1 | 0.36413 | 0.20584 |
| FAM65B | 0.39579 | 0.66372 |
| SGPP2 | 0.39823 | 0.66246 |
| SLC4A8 | 0.45682 | 0.38202 |
| LEF1-AS1 | 0.47215 | 0.70071 |
| RXRA | 0.5708 | 0.37458 |
| NIN | 0.61877 | 0.90267 |

(continued)

| Gene measured | p value by t-test (CR+PR vs PD) | p value by t-test t-test (CR vs PD) |
|---|---|---|
| VCL | 0.6661 | 0.85161 |
| KLRD1 | 0.70258 | 0.85775 |
| EMP3 | 0.71369 | 0.94305 |
| PTPN13 | 0.71717 | 0.24863 |
| AMPD3 | 0.7983 | 0.82587 |
| ICOS | 0.81095 | 0.60306 |
| PASK | 0.81987 | 0.43721 |
| CTBP2 | 0.83499 | 0.59914 |
| HNRPLL | 0.86052 | 0.58529 |
| PDCD1 | 0.89903 | 0.89404 |
| SIRPG | 0.96746 | 0.71762 |
| GBP1 | 0.96752 | 0.2051 |
| ST8SIA1 | 0.98968 | 0.86587 |
| CR: Complete response | | |
| PR: Partial response | | |
| PD: Progressive disease | | |

Whether the sensitivity of a cancer or a tumor to a treatment with a PD-1 immune checkpoint inhibitor can be predicted was checked by gradually increasing the number of genes. The results were as shown in Figure 5A to Figure 5H. As shown in Figure 5A to Figure 5H, when the number of genes were gradually increased from 2 to 10, it was found that the sensitivity of a cancer or a tumor to a treatment with a PD-1 immune checkpoint inhibitor can be predicted with a high degree of accuracy in every case.

Furthermore, whether the sensitivity of a cancer or a tumor to a treatment with a PD-1 immune checkpoint inhibitor can be predicted by using another gene signature was checked. The results were as shown in Figures 6 and 7. As shown in Figures 6 and 7, the sensitivity of a cancer or a tumor to a treatment with a PD-1 immune checkpoint inhibitor was successfully and precisely predicted by using the gene sets shown there as a signature.

It is considered that an anti PD-1 antibody and an anti PD-L1 antibody activate an autoimmune response to a cancer by releasing suppression of an immune response by killer T cells (CD8 positive T cells). In this Example, a gene associated with PD-1 expression of T cells in a tumor tissue and a gene determining the sensitivity to a treatment with an anti PD-1 drug in a tumor tissue were obtained, and then, the sensitivity of a patient to a treatment with a PD-1 immune checkpoint inhibitor was evaluated with a high degree of accuracy.

Additional prediction based on biopsy data

Further, to confirm reproducibility, using different malignant melanoma biopsy data, a gene signature score per patient was calculated. More specifically, using malignant melanoma biopsy data (GSE96619) obtained from the GEO, a gene signature score was calculated. The malignant melanoma biopsy data (GSE96619) contains patient's data before and after a treatment with an immune checkpoint inhibitor (anti PD-1 antibody) and has data of the sensitivity to a treatment with an anti PD-1 antibody. In this Example, a patient who showed the sensitivity and "complete response" or "partial response" as a result of a treatment was defined as "responder (positive)" or "R"; whereas, a patient who showed tolerability or resistance and "progressive disease" was defined as "non-responder (negative)" or "NR." Of them, the relationship between a score calculated from gene signature A based on biopsy data before a treatment with an antibody or a score calculated from gene signature A + B and the sensitivity to a treatment with an anti PD-1 antibody was obtained. The results were as shown in Figure 8A and Figure 8B.

Figure 8A shows the relationship between gene signature scores calculated from gene signature A in individual patients and the sensitivity to a treatment with an anti PD-1 antibody. As shown in Figure 8A, in a patient being "non-responder (negative)" with respect to the sensitivity to a treatment with an anti PD-1 antibody, a score calculated from gene signature A was low (for example, score was 0 or less); whereas in a patient being "responder (positive)" with respect to the sensitivity to a treatment, a score calculated from gene signature A was high.

Figure 8B shows the relationship between gene signature scores calculated from gene signature A + B in individual patients and the sensitivity to a treatment with an anti PD-1 antibody. As shown in Figure 8B, in a patient being "non-responder (negative)" with respect to the sensitivity to a treatment with an anti PD-1 antibody, a score calculated from gene signature A + B was low (for example, score was 0 or less); whereas in a patient being "responder (positive)" with respect to the sensitivity, a score calculated from gene signature A + B was high.

In both gene signatures, it was confirmed that prognosis for the sensitivity to a treatment with an anti PD-1 antibody can satisfactorily be predicted.

Then, whether the sensitivity of a cancer or a tumor to a treatment with a PD-1 immune checkpoint inhibitor can be predicted even by a single gene using malignant melanoma biopsy data (GSE96619) was checked. The results were as shown in Table 7.

[Table 7]

Table 7: Evaluation of sensitivity to treatment with immune checkpoint inhibitor based on single-gene measurement using biopsy sample before treatment with antibody

| Gene measured | GSE96619 (before treatment with antibody) |
| --- | --- |
| | p value by t-test t-test (R vs NR) |
| TBL1X | 0.99563 |
| FAM101B | NA |
| TBC1D4 | 0.98822 |
| PRR5 | 0.62936 |
| PTGDS | 0.10612 |
| FAM211B | NA |
| C17orf67 | 0.33473 |
| RAB11FIP5 | 0.82765 |
| GCNT2 | 0.06574 |
| TSPAN32 | 0.28995 |
| MYB | 0.07897 |
| ADAM8 | 0.07040 |
| ASAP1 | 0.01661 |
| ITGB1 | 0.41219 |
| MBOAT1 | 0.36754 |
| ASB2 | NA |
| COTL1 | 0.33970 |
| FAM65B | 0.39576 |
| SGPP2 | 0.31387 |
| SLC4A8 | 0.18821 |
| LEF1-AS1 | 0.27627 |
| RXRA | 0.00624 |
| NIN | 0.49541 |
| VCL | 0.73152 |
| KLRD1 | 0.59521 |
| EMP3 | 0.33433 |
| PTPN13 | 0.19480 |
| AMPD3 | 0.16364 |
| ICOS | 0.32299 |
| PASK | 0.47154 |
| CTBP2 | 0.01235 |
| HNRPLL | 0.78083 |

(continued)

| Gene measured | GSE96619 (before treatment with antibody) |
|---|---|
| | p value by t-test t-test (R vs NR) |
| PDCD1 | NA |
| SIRPG | 0.99178 |
| GBP1 | 0.71406 |
| ST8SIA1 | 0.07012 |

R: Responder (positive)
NR: Non-Responder (negative)
NA: Not analyzed

As shown in Table 7, it was observed that the expression level of each of the genes has a positive correlation with the sensitivity to a treatment with an immune checkpoint inhibitor. From this, it was apparent that the sensitivity to a treatment with an immune checkpoint inhibitor can be evaluated even by using only one gene.

Note that, Table 7 shows p value obtained by t-test on the relationship between sensitive-to-treatment (R) vs resistance-to-treatment (NR) (R vs NR) to a treatment with an anti PD-1 antibody and evaluation results based on gene expression values with respect to GSE96619 (before treatment with antibody).

As described above, even in a patient's sample of GSE96619 (before treatment with antibody), a big difference in gene expression value was observed between sensitive-to-treatment (R) and resistance-to-treatment (NR). Representative examples showing the relationship between the expression levels of a predetermined single gene in individual patients and "responder (positive)" and "non-responder (negative)" were as shown in Figures 9A to 9H.

The gene signature derived from T cells and defined in this Example can predict the sensitivity to a treatment with a PD-1 immune checkpoint inhibitor. The prediction results were well matched with prediction results of the sensitivity to a treatment with an anti PD-L1 antibody predicted from gene expression data in patient-derived tumor biopsy tissue samples.

It is known that killer T cells invade into a tumor tissue. Thus, analysis using genes contained in the gene set herein and analysis using a gene signature are useful to evaluate the sensitivities of various types of tumors to a treatment with a PD-1 immune checkpoint inhibitor.

It was demonstrated that the gene signature derived from T cells and defined in this Example can be analyzed by isolating T cells from a subject; however, a biopsy tissue sample can be used as an analysis target for predicting the sensitivity of a cancer or a tumor to a treatment with an immune checkpoint inhibitor against PD-1 (for example, anti PD-1 drug such as an anti PD-1 antibody and an anti PD-L1 antibody).

**Claims**

1. A method for predicting sensitivity of a cancer or a tumor of a subject to a treatment with a PD-1 immune checkpoint inhibitor, comprising measuring an expression level(s) of one or more genes selected from the group consisting of the following genes:
ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B, GBP1, FAM211B, PRR5, C17orf67, FAM101B and TBL1X in a sample obtained from the subject.

2. The method according to Claim 1, comprising measuring expression levels of two or more genes selected from the group.

3. The method according to Claim 1, comprising measuring an expression level(s) of at least one or more genes selected from TBL1X, FAM101B, TBC1D4, PRR5 and PTGDS.

4. The method according to Claim 3, comprising measuring an expression level of a TBL1X gene and an expression level(s) of 1, 2, 3, 4, 5, 6, 7 or 8 or more genes selected from the group consisting of ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B, GBP1, FAM211B, PRR5, C17orf67 and FAM101B.

5. The method according to Claim 4, comprising measuring expression levels of at least both TBL1X and FAM101B genes.

6. The method according to Claim 5, comprising measuring expression levels of at least TBL1X, PTGDS, FAM101B, FAM211B, TBC1D4, PRR5 and C17orf67 genes.

7. The method according to any one of Claims 1 to 6, wherein the sample obtained from the subject is a biopsy tissue sample of a cancer or a tumor, or a biopsy tissue sample of a tissue suspected as being a cancer or a tumor.

8. The method according to Claim 7, wherein an expression level of at least a PDCD1 gene is measured.

9. The method according to any one of Claims 1 to 8, wherein the treatment with a PD-1 immune checkpoint inhibitor is administration of an anti PD-1 antibody or an anti PD-L1 antibody.

10. A pharmaceutical composition which is the following:

(i) a pharmaceutical composition comprising an anti PD-1 antibody or an anti PD-L1 antibody, for use in treating or preventing the cancer or the tumor in a subject having a cancer or a tumor predicted to be sensitive to a treatment with a PD-1 immune checkpoint inhibitor by the method according to Claim 1,;
(ii) a pharmaceutical composition comprising an anti PD-1 antibody or an anti PD-L1 antibody, for use in treating or preventing the cancer or the tumor in a subject having a cancer or a tumor predicted to be sensitive to a treatment with a PD-1 immune checkpoint inhibitor by the method according to Claim 2;
(iii) a pharmaceutical composition comprising an anti PD-1 antibody or an anti PD-L1 antibody, for use in treating or preventing the cancer or the tumor in a subject having a cancer or a tumor predicted to be sensitive to a treatment with a PD-1 immune checkpoint inhibitor by the method according to Claim 3;
(iv) a pharmaceutical composition comprising an anti PD-1 antibody or an anti PD-L1 antibody, for use in treating or preventing the cancer or the tumor in a subject having a cancer or a tumor predicted to be sensitive to a treatment with a PD-1 immune checkpoint inhibitor by the method according to Claim 4;
(v) a pharmaceutical composition comprising an anti PD-1 antibody or an anti PD-L1 antibody , for use in treating or preventing the cancer or the tumor in a subject having a cancer or a tumor predicted to be sensitive to a treatment with a PD-1 immune checkpoint inhibitor by the method according to Claim 5;
(vi) a pharmaceutical composition comprising an anti PD-1 antibody or an anti PD-L1 antibody , for use in treating or preventing the cancer or the tumor in a subject having a cancer or a tumor predicted to be sensitive to a treatment with a PD-1 immune checkpoint inhibitor by the method according to Claim 6;
(vii) a pharmaceutical composition comprising an anti PD-1 antibody or an anti PD-L1 antibody , for use in treating or preventing the cancer or the tumor in a subject having a cancer or a tumor predicted to be sensitive to a treatment with a PD-1 immune checkpoint inhibitor by the method according to Claim 7;
(viii) a pharmaceutical composition comprising an anti PD-1 antibody or an anti PD-L1 antibody , for use in treating or preventing the cancer or the tumor in a subject having a cancer or a tumor predicted to be sensitive to a treatment with a PD-1 immune checkpoint inhibitor by the method according to Claim 8; or
(ix) a pharmaceutical composition comprising an anti PD-1 antibody or an anti PD-L1 antibody , for use in treating or preventing the cancer or the tumor in a subject having a cancer or a tumor predicted to be sensitive to a treatment with a PD-1 immune checkpoint inhibitor by the method according to Claim 9.

11. A gene signature comprising one or more genes selected from the group consisting of ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B, GBP1, FAM211B, PRR5, C17orf67, FAM101B and TBL1X, for use in predicting whether or not a cancer or a tumor is sensitive to a treatment with a PD-1 immune checkpoint inhibitor.

12. The gene signature according to Claim 11, comprising at least one or more genes selected from TBL1X, FAM101B, TBC1D4, PRR5 and PTGDS.

13. The gene signature according to Claim 12, comprising at least TBL1X and FAM101B.

14. The gene signature according to Claim 13, comprising at least TBL1X, PTGDS, FAM101B, FAM211B, TBC1D4, PRR5 and C17orf67.

15. A combination of nucleic acid probes or primer sets for a plurality of genes selected from the group consisting of ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B, GBP1, FAM211B, PRR5, C17orf67, FAM101B and TBL1X.

16. The combination of nucleic acid probes or primer sets according to Claim 15, wherein the plurality of genes comprises at least one or more genes selected from TBL1X, FAM101B, TBC1D4, PRR5 and PTGDS.

17. The combination of nucleic acid probes or primer sets according to Claim 16, wherein the plurality of genes comprises at least TBL1X, PTGDS, FAM101B, FAM211B, TBC1D4, PRR5 and C17orf67.

18. A microarray comprising the nucleic acid probes defined in any one of Claims 15 to 17.

19. A kit for use in determining sensitivity to a treatment with a PD-1 immune checkpoint inhibitor, comprising means for measuring an expression level(s) of one or more genes selected from the group consisting of ADAM8, VCL, LEF1-AS1, TSPAN32, CTBP2, RXRA, PTGDS, ITGB1, KLRD1, RAB11FIP5, SLC4A8, NIN, GCNT2, ASB2, HNRPLL, AMPD3, SIRPG, PTPN13, MYB, ASAP1, MBOAT1, COTL1, ICOS, ST8SIA1, PASK, SGPP2, PDCD1, TBC1D4, EMP3, FAM65B, GBP1, FAM211B, PRR5, C17orf67, FAM101B and TBL1X.

20. The kit according to Claim 19, wherein the one or more genes comprise at least one or more genes selected from TBL1X, FAM101B, TBC1D4, PRR5 and PTGDS.

21. The kit according to Claim 20, wherein the one or more genes comprise at least TBL1X, PTGDS, FAM101B, FAM211B, TBC1D4, PRR5 and C17orf67.

# FIG. 1

PATIENTS (IN THE DESCENDING ORDER OF SCORE)

# FIG. 2

PATIENTS (IN THE DESCENDING ORDER OF SCORE)

# FIG. 3

# FIG. 4A

# FIG. 4B

BAR CHART

**FAM101B**

ANTI PD-1 ANTIBODY RESPONSE
COMPLETE RESPONSE (CR)
PARTIAL RESPONSE (PR)
PROGRESSIVE DISEASE (PD)

GENE EXPRESSION LEVEL (Log VALUE)

# FIG. 4C

**TBC1D4**

BAR CHART

ANTI PD-1 ANTIBODY RESPONSE
COMPLETE RESPONSE (CR)
PARTIAL RESPONSE (PR)
PROGRESSIVE DISEASE (PD)

GENE EXPRESSION LEVEL (Log VALUE)

# FIG. 4D

BAR CHART

**PRR5**

ANTI PD-1 ANTIBODY RESPONSE

COMPLETE RESPONSE (CR)
PARTIAL RESPONSE (PR)
PROGRESSIVE DISEASE (PD)

GENE EXPRESSION LEVEL (Log VALUE)

# FIG. 4E

**PTGDS**

BAR CHART

ANTI PD-1 ANTIBODY RESPONSE

COMPLETE RESPONSE (CR)
PARTIAL RESPONSE (PR)
PROGRESSIVE DISEASE (PD)

GENE EXPRESSION LEVEL (Log VALUE)

# FIG. 4F

**FAM211B**

BAR CHART

GENE EXPRESSION LEVEL (Log VALUE)

ANTI PD-1 ANTIBODY RESPONSE
COMPLETE RESPONSE (CR)
PARTIAL RESPONSE (PR)
PROGRESSIVE DISEASE (PD)

# FIG. 4G

**RAB11FIP5**

BAR CHART

GENE EXPRESSION LEVEL (Log VALUE)

ANTI PD-1 ANTIBODY RESPONSE
COMPLETE RESPONSE (CR)
PARTIAL RESPONSE (PR)
PROGRESSIVE DISEASE (PD)

# FIG. 4H

## GCNT2

BAR CHART

GENE EXPRESSION LEVEL (Log VALUE)

ANTI PD-1 ANTIBODY RESPONSE
- COMPLETE RESPONSE (CR)
- PARTIAL RESPONSE (PR)
- PROGRESSIVE DISEASE (PD)

# FIG. 4I

## TSPAN32

BAR CHART

GENE EXPRESSION LEVEL (Log VALUE)

ANTI PD-1 ANTIBODY RESPONSE
- COMPLETE RESPONSE (CR)
- PARTIAL RESPONSE (PR)
- PROGRESSIVE DISEASE (PD)

# FIG. 4J

BAR CHART

**MYB**

ANTI PD-1 ANTIBODY RESPONSE
COMPLETE RESPONSE (CR)
PARTIAL RESPONSE (PR)
PROGRESSIVE DISEASE (PD)

GENE EXPRESSION LEVEL (Log VALUE)

# FIG. 4K

BAR CHART

**ADAM8**

ANTI PD-1 ANTIBODY RESPONSE
COMPLETE RESPONSE (CR)
PARTIAL RESPONSE (PR)
PROGRESSIVE DISEASE (PD)

GENE EXPRESSION LEVEL (Log VALUE)

# FIG. 4L

## ASAP1

# FIG. 4M

## ITGB1

# FIG. 4N

**BAR CHART**

## MBOAT1

ANTI PD-1 ANTIBODY RESPONSE

COMPLETE RESPONSE (CR)
PARTIAL RESPONSE (PR)
PROGRESSIVE DISEASE (PD)

GENE EXPRESSION LEVEL (Log VALUE)

# FIG. 4O

**BAR CHART**

## ASB2

ANTI PD-1 ANTIBODY RESPONSE

COMPLETE RESPONSE (CR)
PARTIAL RESPONSE (PR)
PROGRESSIVE DISEASE (PD)

GENE EXPRESSION LEVEL (Log VALUE)

# FIG. 4P

## COTL1

BAR CHART

GENE EXPRESSION LEVEL (Log VALUE)

ANTI PD-1 ANTIBODY RESPONSE
COMPLETE RESPONSE (CR)
PARTIAL RESPONSE (PR)
PROGRESSIVE DISEASE (PD)

# FIG. 4Q

BAR CHART

## SGPP2

GENE EXPRESSION LEVEL (Log VALUE)

ANTI PD-1 ANTIBODY RESPONSE
COMPLETE RESPONSE (CR)
PARTIAL RESPONSE (PR)
PROGRESSIVE DISEASE (PD)

# FIG. 4R

BAR CHART

**FAM65B**

ANTI PD-1 ANTIBODY RESPONSE
COMPLETE RESPONSE (CR)
PARTIAL RESPONSE (PR)
PROGRESSIVE DISEASE (PD)

GENE EXPRESSION LEVEL (Log VALUE)

# FIG. 4S

BAR CHART

**SLC4A8**

ANTI PD-1 ANTIBODY RESPONSE
COMPLETE RESPONSE (CR)
PARTIAL RESPONSE (PR)
PROGRESSIVE DISEASE (PD)

GENE EXPRESSION LEVEL (Log VALUE)

# FIG. 4T

BAR CHART

## LEF1-AS1

ANTI PD-1 ANTIBODY RESPONSE
- COMPLETE RESPONSE (CR)
- PARTIAL RESPONSE (PR)
- PROGRESSIVE DISEASE (PD)

# FIG. 4U

BAR CHART

## RXRA

ANTI PD-1 ANTIBODY RESPONSE
- COMPLETE RESPONSE (CR)
- PARTIAL RESPONSE (PR)
- PROGRESSIVE DISEASE (PD)

# FIG. 4V

## NIN

BAR CHART

GENE EXPRESSION LEVEL (Log VALUE)

ANTI PD-1 ANTIBODY RESPONSE
COMPLETE RESPONSE (CR)
PARTIAL RESPONSE (PR)
PROGRESSIVE DISEASE (PD)

# FIG. 4W

BAR CHART

## VCL

GENE EXPRESSION LEVEL (Log VALUE)

ANTI PD-1 ANTIBODY RESPONSE
COMPLETE RESPONSE (CR)
PARTIAL RESPONSE (PR)
PROGRESSIVE DISEASE (PD)

# FIG. 4X

BAR CHART

## KLRD1

ANTI PD-1 ANTIBODY RESPONSE
COMPLETE RESPONSE (CR)
PARTIAL RESPONSE (PR)
PROGRESSIVE DISEASE (PD)

GENE EXPRESSION LEVEL (Log VALUE)

# FIG. 4Y

BAR CHART

## EMP3

ANTI PD-1 ANTIBODY RESPONSE
COMPLETE RESPONSE (CR)
PARTIAL RESPONSE (PR)
PROGRESSIVE DISEASE (PD)

GENE EXPRESSION LEVEL (Log VALUE)

# FIG. 4Z

## PTPN13

BAR CHART

ANTI PD-1 ANTIBODY RESPONSE
COMPLETE RESPONSE (CR)
PARTIAL RESPONSE (PR)
PROGRESSIVE DISEASE (PD)

GENE EXPRESSION LEVEL (Log VALUE)

# FIG. 4AA

## ICOS

BAR CHART

ANTI PD-1 ANTIBODY RESPONSE
COMPLETE RESPONSE (CR)
PARTIAL RESPONSE (PR)
PROGRESSIVE DISEASE (PD)

GENE EXPRESSION LEVEL (Log VALUE)

# FIG. 4BB

## AMPD3

BAR CHART

GENE EXPRESSION LEVEL (Log VALUE)

ANTI PD-1 ANTIBODY RESPONSE
- COMPLETE RESPONSE (CR)
- PARTIAL RESPONSE (PR)
- PROGRESSIVE DISEASE (PD)

# FIG. 4CC

## PASK

BAR CHART

GENE EXPRESSION LEVEL (Log VALUE)

ANTI PD-1 ANTIBODY RESPONSE
- COMPLETE RESPONSE (CR)
- PARTIAL RESPONSE (PR)
- PROGRESSIVE DISEASE (PD)

# FIG. 4DD

## CTBP2

BAR CHART

GENE EXPRESSION LEVEL (Log VALUE)

ANTI PD-1 ANTIBODY RESPONSE
COMPLETE RESPONSE (CR)
PARTIAL RESPONSE (PR)
PROGRESSIVE DISEASE (PD)

# FIG. 4EE

## HNRPLL

BAR CHART

GENE EXPRESSION LEVEL (Log VALUE)

ANTI PD-1 ANTIBODY RESPONSE
COMPLETE RESPONSE (CR)
PARTIAL RESPONSE (PR)
PROGRESSIVE DISEASE (PD)

# FIG. 4FF

## PDCD1

BAR CHART

GENE EXPRESSION LEVEL (Log VALUE)

ANTI PD-1 ANTIBODY RESPONSE
- COMPLETE RESPONSE (CR)
- PARTIAL RESPONSE (PR)
- PROGRESSIVE DISEASE (PD)

# FIG. 4GG

## GBP1

BAR CHART

GENE EXPRESSION LEVEL (Log VALUE)

ANTI PD-1 ANTIBODY RESPONSE
- COMPLETE RESPONSE (CR)
- PARTIAL RESPONSE (PR)
- PROGRESSIVE DISEASE (PD)

# FIG. 4HH

BAR CHART

**SIRPG**

ANTI PD-1 ANTIBODY RESPONSE
COMPLETE RESPONSE (CR)
PARTIAL RESPONSE (PR)
PROGRESSIVE DISEASE (PD)

GENE EXPRESSION LEVEL (Log VALUE)

4.5
4
3.5
3
2.5
2
1.5
1
0.5
0

# FIG. 4II

BAR CHART

**ST8SIA1**

ANTI PD-1 ANTIBODY RESPONSE
COMPLETE RESPONSE (CR)
PARTIAL RESPONSE (PR)
PROGRESSIVE DISEASE (PD)

GENE EXPRESSION LEVEL (Log VALUE)

3.5
3
2.5
2
1.5
1
0.5
0

# FIG. 5A

BAR CHART

Gene signature: TBL1X + FAM101B

# FIG. 5B

BAR CHART

Gene signature: TBL1X + FAM101B + TBC1D4

# FIG. 5C

BAR CHART  Gene signature: TBL1X + FAM101B + TBC1D4 + PRR5

ANTI PD-1 ANTIBODY RESPONSE
COMPLETE RESPONSE (CR)
PARTIAL RESPONSE (PR)
PROGRESSIVE DISEASE (PD)

# FIG. 5D

Gene signature: TBL1X + FAM101B + TBC1D4 + PRR5 + PTGDS

BAR CHART

ANTI PD-1 ANTIBODY RESPONSE
COMPLETE RESPONSE (CR)
PARTIAL RESPONSE (PR)
PROGRESSIVE DISEASE (PD)

# FIG. 5E

**Gene signature: TBL1X + FAM101B + TBC1D4 + PRR5 + PTGDS + RAB11FIP5**

BAR CHART

ANTI PD-1 ANTIBODY RESPONSE
COMPLETE RESPONSE (CR)
PARTIAL RESPONSE (PR)
PROGRESSIVE DISEASE (PD)

# FIG. 5F

**Gene signature: TBL1X + FAM101B + TBC1D4 + PRR5 + PTGDS + RAB11FIP5 + GCNT2**

BAR CHART

ANTI PD-1 ANTIBODY RESPONSE
COMPLETE RESPONSE (CR)
PARTIAL RESPONSE (PR)
PROGRESSIVE DISEASE (PD)

# FIG. 5G

BAR CHART Gene signature: TBL1X + FAM101B + TBC1D4 + PRR5 + PTGDS + RAB11FIP5 + GCNT2 + FAM211B

# FIG. 5H

BAR CHART Gene signature: TBL1X + FAM101B + TBC1D4 + PRR5 + PTGDS + RAB11FIP5 + GCNT2 + FAM211B + TSPAN32

# FIG. 5I

BAR CHART  Gene signature: TBL1X + FAM101B + TBC1D4 + PRR5
+ PTGDS + RAB11FIP5 + GCNT2 + FAM211B
+ TSPAN32 + MYB

# FIG. 6

BAR CHART  Gene signature: PTGDS, TBC1D4, FAM211B, PRR5,
C17orf67, FAM101B, AND TBL1X

# FIG. 7

BAR CHART **Gene signature: FAM211B, PRR5, C17orf67, FAM101B, AND TBL1X**

ANTI PD-1 ANTIBODY RESPONSE
COMPLETE RESPONSE (CR)
PARTIAL RESPONSE (PR)
PROGRESSIVE DISEASE (PD)

# FIG. 8A

## GENE SIGNATURE A

ANTI PD-1 ANTIBODY RESPONSE
Responder (POSITIVE)
Non-responder (NEGATIVE)

# FIG. 8B

GENE SIGNATURE A + B

ANTI PD-1 ANTIBODY RESPONSE
Responder (POSITIVE)
Non-responder (NEGATIVE)

# FIG. 9A

## RXRA

ANTI PD-1 ANTIBODY RESPONSE
Responder (POSITIVE)
Non-responder (NEGATIVE)

# FIG. 9B

## CTBP2

# FIG. 9C

## ASAP1

# FIG. 9D

## GCNT2

# FIG. 9E

## ST8SIA1

# FIG. 9F

## ADAM8

# FIG. 9G

## MYB

# FIG. 9H

## PTGDS

## INTERNATIONAL SEARCH REPORT

| International application No. |
| --- |
| PCT/JP2017/041953 |

**A. CLASSIFICATION OF SUBJECT MATTER**
Int. Cl.    C12Q1/68(2018.01)i, A61K39/395(2006.01)i, A61P35/00(2006.01)i,
C07K16/28(2006.01)i, C12N15/09(2006.01)i, G01N33/50(2006.01)i,
G01N33/68(2006.01)i, G01N37/00(2006.01)i
According to International Patent Classification (IPC) or to both national classification and IPC

**B. FIELDS SEARCHED**

Minimum documentation searched (classification system followed by classification symbols)
Int. Cl.    C12Q1/68, A61K39/395, A61P35/00, C07K16/28, C12N15/09, G01N33/50,
G01N33/68, G01N37/00

Documentation searched other than minimum documentation to the extent that such documents are included in the fields searched
Published examined utility model applications of Japan      1922-1996
Published unexamined utility model applications of Japan    1971-2018
Registered utility model specifications of Japan            1996-2018
Published registered utility model applications of Japan    1994-2018

Electronic data base consulted during the international search (name of data base and, where practicable, search terms used)
JSTPlus/JMEDPlus/JST7580 (JDreamIII), CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| Y | WO 2016/094377 A1 (MERCK SHARP & DOHME CORP.) 16 June 2016, claims, page 2, line 5 to page 4, line 32, table 1, examples & EP 3230498 A1 | 1-21 |
| Y | WO 2016/044207 A1 (THE JOHNS HOPKINS UNIVERSITY) 24 March 2016, claims, examples & US 2017/0275705 A1 | 1-21 |

☒ Further documents are listed in the continuation of Box C.      ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| | |

| Name and mailing address of the ISA/ <br>    Japan Patent Office <br>    3-4-3, Kasumigaseki, Chiyoda-ku, <br>    Tokyo 100-8915, Japan | Authorized officer <br><br><br> Telephone No. |
| --- | --- |

Form PCT/ISA/210 (second sheet) (January 2015)

**INTERNATIONAL SEARCH REPORT**

| International application No. |
|---|
| PCT/JP2017/041953 |

C (Continuation).   DOCUMENTS CONSIDERED TO BE RELEVANT

| Category* | Citation of document, with indication, where appropriate, of the relevant passages | Relevant to claim No. |
|---|---|---|
| Y | 小西郁夫ほか,がん免疫逃避機構を標的にした次世代型免疫治療の臨床応用と新規バイオマーカーの探索に関する研究,厚生労働科学研究費補助金 難病・がん等の疾患分野の医療の実用化研究事業(がん関係研究分野)がん免疫逃, pp. 1-8, 2014 non-official translation (KONISHI, Ikuo et al., Research relating to clinical applications of next-generation immunotherapy targeting cancer immune evasion mechanisms and the search for novel biomarkers, Ministry of Health, Labour and Welfare Research Grant, practical research of treatments for malignant diseases, cancer etc. (cancer related research) immune escape in cancer) | 1-21 |
| X<br>Y<br>A | JP 2014-158500 A (ANSELM INST NAT DE LA SANTE ET DE LA RECH MEDICALE) 04 September 2014, claims, embodiments & US 2009/0215053 A1, claims, examples & WO 2007/045996 A1 & EP 1777523 A1 | 11-14<br>15-18<br>1-10, 19-21 |
| X<br>Y<br>A | MIZUHASHI, K., et al., "Accession:NP_874364.1[gi: 359845], Definition: filamin-interacting protein FAM101B [Homosapiens].", NCBI Protein, 12 August 2016 uploaded, [retrieved on 15 February 2018] Retrieved from the Internet: 〈URL http://www.ncbi.nih.gov./protein/33186901?sat=4&satkey=170639756> | 11-14<br>15-18<br>1-10, 19-21 |
| X<br>Y<br>A | STOY, C., et al.,"Accession:NP_005638 [gi: 6907], Definition: F-box-like/WD repeat-containing protein TBLXI isoform a [Homosapiens].", 26 August 2016 uploaded, [retrieved on 15 February 2018] retrieved from the Internet= 〈URL, http://www.ncbi.nih.gov/protein/5032159?sat=4&satkey=17267840> | 11-14<br>15-18<br>1-10, 19-21 |
| A | WO 2016/175275 A1 (KYOTO UNIVERSITY) 03 November 2016, entire text (Family: none) | 1-21 |
| P, A | WO 2017/065000 A1 (KYOTO UNIVERSITY) 20 April 2017, entire text (Family: none) | 1-21 |
| P, A | HUANG C. Alexander, et al., T-cell invigoration to tumour burden ratio associated with anti-PD-1 response., NATURE, 04 April 2017, vol. 545, pp. 60-77 | 1-21 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Immunity,* 2013, vol. 39 (1), 1-10
- *Cancer Discov,* 2016, vol. 6 (7), 703-713
- *N Engl J Med.,* 2012, vol. 366 (26), 2443-2454

- **BOHNSACK O. et al.** *Ann. Oncl.,* 2014, vol. 25 (4), 361-372
- **MARTIN et al.** *BMC Systems Biology,* 2012, vol. 6, 54